# EUROPEAN PATENT APPLICATION

(11) **EP 1 132 381 A1**
(43) Date of publication of application: **12.09.2001**
(21) Application number: 00104916.2
(22) Date of filing: 08.03.2000
(51) Int. Cl.: C07D 235/12, C07D 471/04, A61K 31/4184, A61K 31/4188

(54) **Ester derivatives of dimethylpropionic acid and pharmaceutical compositions containing them**

(71) Applicant: CERMOL S.A., CH-1902 Evionnaz (CH)
(72) Inventor: Statkow, Pierre, 1203 Geneve (CH); Straumann, Danielle, 1920 Martigny (CH); Chatterjee, Shyam, 76139 Karlsruhe (DE); Alvarez-Builla Gomez, Julio, 28033 Madrid (ES); Sunkel Letelier, Carlos, 28033 Madrid (ES); Fau De Casa-Juana Munoz, Miguel, 28043 Madrid (ES); Minguez Ortega, José M., 28820 Coslada (ES); Paz Matia Martin, M., 28005 Madrid (ES)
(74) Representative: KIRKER & Cie S.A.

(57) **Abstract**

The present invention relates to esters of 2,2-dimethylpropionic acid having the general formula (I) 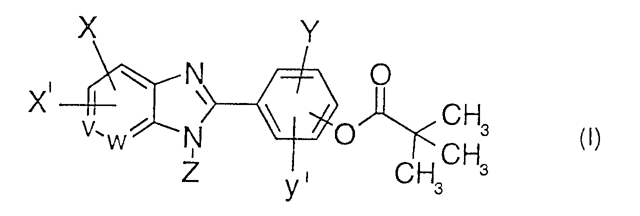 or pharmacological acceptable salts thereof, as well as to pharmaceutical compositions containing said compounds and having an inhibitory activity of elastase.

## Description

The present invention relates to new esters of 2,2-dimethylpropionic acids, to the use thereof as agents having an inhibitory activity of elastase and to pharmaceutical compositions containing these compounds or a pharmaceutically cceptable salt thereof.
More particularly, the object of the invention consists in compounds of general formula (I), 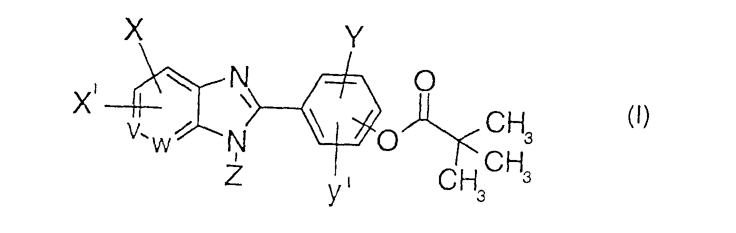 or a pharmaceutically acceptable salt thereof, where
x and x' represent a hydrogen atom, an alkyl group in C1-C4, an halogen atom or a group nitro;
y and y' represent a hydrogen atom, a group alkyl in C1-C4, a group alkoxy in C1-C4, an halogen atom or a group dialkyl(C1-C4)amino;
z represents a hydrogen atom, a dialkyl(C1-C4)aminoalkyl(C1-C4) group or a piperidinyl-alkyl(C1-C4) group; and
v and w represent a carbon atom bound to a hydrogen atom (CH) or a nitrogen atom substituted or not.
   More particularly, in the above formula (I), the definition of the substituents may be the following:
x and/or x' represent the group methyl or nitro, or a chlorine atom;
y and/or y' represent the group methyl, methoxy, nitro or diethylamino, or a chlorine, a bromine or a fluorine atom; and
z represents a group dimethylaminoethyl, dimethylaminopropyl, diisopropylaminoethyl or piperidinyl-ethyl.
   In these compounds of formula (I), v or w may represent a nitrogen atom substituted by a group methyl, ethyl, benzyl, piperidinyl-ethyl, piperidinyl-propyl, bis(fluorophenyl)methyl-piperazinyl-ethyl or bis(fluorophenyl)methyl-piperazinylpropyl.

Some specific examples of the compounds of the present invention, without setting a limit to it, are the followings:
2,2-Dimethyl-propionic acid 4-(1H-benzimidazol-2-yl)-phenyl ester
2,2-Dimethyl-propionic acid 4-(1H-benzimidazol-2-yl)-2-ethoxy-phenyl ester
2,2-Dimethyl-propionic acid 4-(1H-benzimidazol-2-yl)-2,6-dimethoxy-phenyl ester
2,2-Dimethyl-propionic acid 4-(1H-benzimidazol-2-yl)-2-chloro-phenyl ester
2,2-Dimethyl-propionic acid 4-(1H-benzoimidazol-2-yl)-2-nitro-phenyl ester
2,2-Dimethyl-propionic acid 4-(1H-benzimidazol-2-yl)-2-nitro-6-methoxy-phenyl ester
2,2-Dimethyl-propionic acid 4-(5-chloro-1 H-benzimidazol-2-yl)phenyl ester
2,2-Dimethyl-propionic acid 4-(5-chloro-1 H-benzimidazol-2-yl)-2-methoxy-phenyl ester
2,2-Dimethyl-propionic acid 4-(5,6-dimethyl-1H-benzimidazol-2-yl)phenyl ester
2,2-Dimethyl-propionic acid 4-(5-methyl-1 H-benzimidazol-2-yl)phenyl ester
2,2-Dimethyl-propionic acid 4-(5-methyl-1 H-benzimidazol-2-yl)-2-methoxy-phenyl ester
2,2-Dimethyl-propionic acid 4-(5,6-dimethyl-1 H-benzimidazol-2-yl)-2-methoxyphenyl ester
2,2-Dimethyl-propionic acid 4-(5-nitro-1 H-benzimidazol-2-yl)phenyl ester
2,2-Dimethyl-propionic acid 4-(5-nitro-1 H-benzimidazol-2-yl)-6-methoxy-2-nitrophenyl ester
2,2-Dimethylpropionic acid 4-[1-(2-dimethylaminoethyl)-1 H-benzimidazol-2-yl] phenyl ester.
2,2-Dimethylpropionic acid 2-bromo-4-[1-(2-dimethylaminoethyl)-1H-benzimidazol-2-yl]phenyl ester
2,2-Dimethylpropionic acid 4-[1-(2-dimethylaminopropyl)-1 H-benzimidazol-2-yl]phenyl ester, dihydrogen oxalate
2,2-Dimethylpropionic acid 4-[1-(2-diisopropylaminoethyl)-1H-benzimidazol-2-yl]phenyl ester.
2,2-Dimethylpropionic acid 4-[5,6-dichloro-1-(2-dimethylaminoethyl) 1H-benzimidazol-2-yl] phenyl ester
2,2-Dimethylpropionic acid 4-[5,6-dimethyl-3-(2-piperidin-1-yl-ethyl)-1H-benzimidazol-2-yl] phenyl ester
2,2-Dimethylpropionic acid 2-fluoro-4-[1-(2-piperidin-1-yl ethyl)-1H-benzimidazol-2-yl] phenyl ester
2,2-Dimethyl-propionic acid 2-(1H-benzimidazol-2-yl)phenyl ester
2,2-Dimethyl-propionic acid 2-(1H-benzimidazol-2-yl)-4-chloro-phenyl ester
2,2-Dimethyl-propionic acid 2-(1H-benzimidazol-2-yl)-5-chloro-phenyl ester
2,2-Dimethyl-propionic acid 2-(1H-benzimidazol-2-yl)-4,6-dichloro-phenyl ester
2,2-Dimethyl-propionic acid 2-(1H-benzimidazol-2-yl)-6-methoxy-phenyl ester
2,2-Dimethyl-propionic acid 2-(5-chloro-1 H-benzimidazol-2-yl)phenyl ester
2,2-Dimethyl-propionic acid 2-(-5-chloro-1 H-benzimidazol-2-yl)-5-diethylaminophenyl ester
2,2-Dimethyl-propionic acid 2-(5,6-dimethyl-1 H-benzimidazol-2-yl)phenyl ester
2,2-Dimethyl-propionic acid 2-(5-methyl-1 H-benzimidazol-2-yl)-4-chloro-phenyl ester
2,2-Dimethyl-propionic acid 2-(5,6-dimethyl-1 H-benzimidazol-2-yl)-diethylaminophenyl ester
2,2-Dimethyl-propionic acid 2-(5-nitro-1 H-benzimidazol-2-yl)-phenyl ester
2,2-Dimethyl-propionic acid 2-(5-nitro-1H-benzimidazol-2-yl)-4-chloro-phenyl ester
2,2-Dimethyl-propionic acid 2-(5-nitro-1 H-benzimidazol-2-yl)-6-methyl-phenyl ester
2,2-Dimethyl-propionic acid 5-(1H-benzimidazol-2-yl)-phenyl ester
2,2-Dimethyl-propionic acid 3-(1H-benzimidazol-2-yl)-6-methoxy-phenyl ester
2,2-Dimethyl-propionic acid 3-(1H-benzimidazol-2-yl)-4-nitro-phenyl ester
2,2-Dimethyl-propionic acid 3-(5-chloro-1 H-benzimidazol-2-yl)phenyl ester
2,2-Dimethyl-propionic acid 3-(5,6-dimethyl-1 H-benzimidazol-2-yl)phenyl ester
2,2-Dimethyl-propionic acid 3-(5-nitro-1H-benzimidazol-2-yl)phenyl ester
2,2-Dimethyl-propionic acid 3-(5-nitro-1H-benzimidazol-2-yl)-4-nitro-phenyl ester
2,2-Dimethyl-propionic acid 4-(3H-imidazo[4,5-b]pyridin-2-yl)-phenyl ester
2,2-Dimethyl-propionic acid 4-(3H-imidazo[4,5-b]pyridin-2-yl)-2-methoxy-phenyl ester
2,2-Dimethyl-propionic acid 2-(3H-imidazo[4,5-b]pyridin-2-yl)-phenyl ester
2,2-Dimethyl-propionic acid 3-(3H-imidazo[4,5-b]pyridin-2-yl)-phenyl ester
2,2-Dimethyl-propionic acid 4-(3H-imidazo[4,5-c]pyridin-2-yl)-phenyl ester
2,2-Dimethyl-propionic acid 4-(3H-imidazo[4,5-c]pyridin-2-yl)-2-methoxy-phenyl ester
2,2-Dimethyl-propionic acid 3-(3H-imidazo[4,5-c]pyridin-2-yl)-phenyl ester
2,2-Dimethylpropionic acid 4-(5-methyl-5H-imidazo[4,5-c]pyridin-2-yl) phenyl ester.
2,2-Dimethylpropionic acid 4-(5-ethyl-5H-imidazo[4,5-c]pyridin-2-yl) phenyl ester, hydrogen oxalate
2,2-Dimethylpropionic acid 4-(5-benzyl-5H-imidazo[4,5-c]pyridin-2-yl)phenyl ester
2,2-Dimethylpropionic acid 4-[5-(2-piperidin-1-yl ethyl)-5H-imidazo[4,5-c]pyridin-2-yl] phenyl ester
2,2-Dimethylpropionic acid 4-[5-(2-piperidin-1-yl propyl)-5H-imidazo[4,5-c] pyridin-2-dihydrogen oxalate yl]phenyl ester
2,2-dimethylpropionic acid 4-[5-(3-{4-[bis-(4-fluoro-phenyl)-methyl]-piperazin-1-yl}-ethyl)-5H-imidazo[4,5-c]pyridin-2-yl]-phenyl-ester
2,2-Dimethyl-propionic acid 4-[5-(3-{4-[bis-(4-fluoro-phenyl)-methyl]-piperazin-1-yl}-propyl)-5H-imidazo[4,5-c]pyridin-2-yl]-phenyl ester
2,2-Dimethyl-propionic acid 4-[(1 -H-benzimidazol-2-yl)-2,2-dimethyl-propionyloxy]-phenyl ester

The new compounds can be obtained with usual known methods, which are already described in the literature, for the esterification of phenolic derivatives, with 2,2-dimethylpropionic acid or its corresponding acid chloride or anhydride. In that way, a compound with general formula (II) 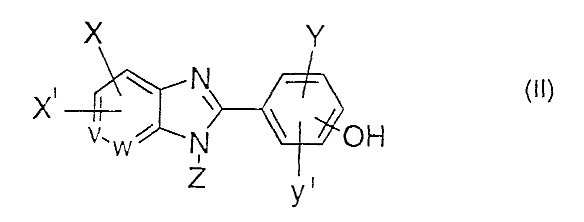 where x, x', y, y', z, v and w are as defined above, is reacted with 2,2-dimethylpropionic acid or its acid chloride or its anhydride to afford a compound with general formula (I).
The methods used for esterification of the general formula (II) compounds, with 2,2-dimethylpropionic acid derivatives can be those described for example in EP patents 0 649 846 or 0 347 168.
More generally, the following methods used to obtain the intermediate compounds with general formula (II) can be mentioned :
- Haugwitz, R.D.; Maurer, B.V.; Jacobs, G.A.; Marayanan, V.L; *J.Med.Chem*., (1979), Vol. 22, No. 9, 1113.
- Yildir, I.; Uzbay, T.; Noyanalpan, N.; *J.Fac. Pharm. Gazi,* vol. 7, No.2,111-24 (1990). - Perginer, H.; Abbasoglu, U.; Noyanalpan, N.; J.Fac. Pharm. Gazi, vol. 7, No. 2,125-40 (1990).
- Kumazawa, T.; Harakawa, H.; Fukui, H. et al.; *Bioorg. Med. Chem. Lett.* Vol. 5, No. 16, 1829-32 (1995).
- Ohalapathy, C.V.; Veeranagaiah, V.; Kondal, K.; Subba Rao, N.V., Indian J. of Chem., vol. 17B, June 1979, 566-8. - Ueda, M.; Sato, M.; Mochizuki, A.; *Macromolecules,* (1985), vol. 18,2723-6.
- Sluka, J.; Novak, J.; Budesinsky, Z.; *Coll. Czeck. Chem. Comun.,* vol. 41,3628-34 (1976).

The pharmacologically acceptable salts produced by addition of acids to the compounds with general formula (I) are prepared in the conventional way, that is through addition to a free base (I) solution or suspension, of one or two equivalents of a pharmacologically acceptable organic or inorganic acid. Examples of acids are : hydrochloric, hydrobromic, sulfuric, phosphoric, acetic, citric, oxalic, malonic, salycilic, malic, lactic, *p*-toluensulphonic, gluconic, fumaric, succinic, ascorbic, maleic, methanesulphonic and benzenesulphonic. The salts afforded by addition of acids can be advantageous, due to some of their physical properties just as high solubility in polar solvents like, for example, water. This would facilitate preparations which include the product administration dissolved in water.
The compounds (I) of the present patent can be used as pharmaceutical agents having an inhibitory activity of elastase, and therefore be administered either solely, or more generally mixed with a pharmacological coadjuvant, chosen in agreement with the administration way and the standard pharmacological practice. For example, they can be administered by oral via in form of either tablets which contain excipients, just as starch or lactose, or capsules, solely or mixed with excipients, or sirups or suspensions which contain colorant or aromatic agents. Also, they can be injected by parenteral via, for example, intramuscular, intravenous or subcutaneously. In the parenteral administration, they can be used preferably in the form of sterile aqueous solution, which can contain another solutes, for example, glucose or any salt, in order to make the solution isotonic.

The pharmacological compositions will be able to contain a quantity of some of the compounds with general formula (I), so that the dose level administrated is comprised between 0,001 and 10 mg/kg. The active principle quantity in each dose form will be comprised approximately between 0.05 and 1 mg or between 0.1 and 99% by weight of the preparation, preferably between 2 and 50% by weight for oral preparations. The active substance dose per day depends on the administration form. In general, between 50 and 100mg/day are administered by oral via. While the intramuscular administration can be provided in a single dose or divided in up to 3 doses, the intravenous administration can include a dropper for its dosification in continuous. Necessarily, there will be variations which would depend on the weight and subject conditions to be treated and the particular administration via.

The following examples illustrate the present invention without setting limits to it:

### Example 1

### 2,2-Dimethyl-propionic acid 4-(1H-benzimidazol-2-yl)-phenyl ester

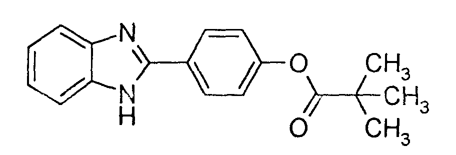

Initially, 35 mL of triethylamine were added dropwise to a stirred solution of 20 g (0.095 mol) of 2-(4-hydroxyphenyl)benzimidazole in 115 mL of anhydrous CH₂Cl₂, using external cooling with an ice-water bath. Then, 11.47 g (0.095 mol) of 2, 2-dimethylpropionyl chloride were dropwise added. Once the adition was completed, the resultant mixture was stirred at about 0°C for 30 minutes and then, at room temperature for 4 additional hours. Finally, 100 mL of ethyl eter were added to the reaction mixture, the insoluble residue was filtered off, and the remaining liquid was washed with H₂O (2 x 250 ml). Then, the organic phase was dried over anhydrous Na₂SO₄. Then, after evaporating the solvent under reduced pressure, the product was isolated as a white solid with m.p. 308-10°C (recrystallized in ethanol) with a yield of 85 %.

| **Quantitative Analysis**: Calculated for C₁₈H₁₈N₂O₂ | | | |
|---|---|---|---|
| | % C | % H | % N |
| Calculated | 73.45 | 6.16 | 9.52 |
| Found | 73.34 | 6.37 | 9.31 |

### Example 2

### 2,2-Dimethyl-propionic acid 4-(1H-benzimidazol-2-yl)-2-ethoxy-phenyl ester

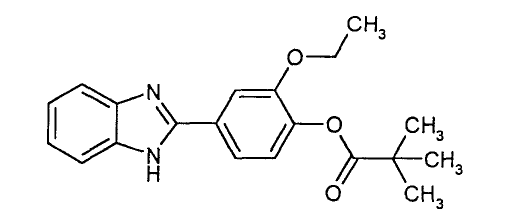

Initially, 14 mL of triethylamine were added dropwise to a stirred solution of 10 g (0.039 mol) of 2-(3-ethoxy-4-hydroxyphenyl)benzimidazole in 47 mL of anhydrous CH₂Cl₂, using external cooling with an ice-water bath, and then, 4.74 g (0.039 mol) of 2, 2-dimethylpropionyl chloride were added. Once the addition was completed, the resultant mixture was stirred at about O°C for 30 minutes and then, at room temperature for 7 hours. At the end, 75 mL of ethyl ether were added to the reaction mixture, the insoluble residue was filtered off, and the liquid was washed with H₂O (2 x 100 mL). Then, the organic phase was dried over anhydrous Na₂SO₄, the solvent was evaporated under reduced pressure, and the product was isolated as a solid with m.p. 180-1°C (recrystallized in ethanol) with a yield of 52%.

| **Quantitative Analysis:** Calculated for C₂₀H₂₂N₂O₃ | | | |
|---|---|---|---|
| | % C | % H | % N |
| Calculated | 70.99 | 6.55 | 8.28 |
| Found | 70.69 | 6.61 | 8.07 |

### Example 3

### 2,2-Dimethyl-propionic acid 4-(1H-benzimidazol-2-yl)-2,6-dimethoxy-phenyl ester

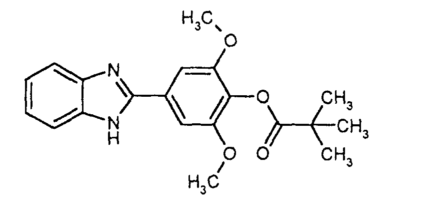

Initially, 7 mL of triethylamine were added dropwise to a stirred solution composed of 5 g (0.018 mol) of 2-(3,5-dimethoxy-4-hydroxyphenyl)benzimidazole in 23 mL of anhydrous CH₂Cl₂, using external cooling with an ice-water bath, and next, 2.23 g (0.018 mol) of 2, 2-dimethylpropionyl chloride were added. Once the addition was completed, the resultant mixture was stirred at about O°C for 30 minutes and then, at room temperature for 6 hours. Finally, 40 mL of ethyl ether were added to the reaction mixture, the insoluble residue was filtered off, and the liquid was washed with H₂O (2 x 100 mL). Then, the organic phase was dried over anhydrous Na₂SO₄, the solvent removed under reduced pressure, and the product was obtained as a solid with m.p. 243-5 °C (recrystallized in methanol) with a yield of 45%.

| **Quantitative Analysis:** Calculated for C₂₀H₂₂N₂O₄ | | | |
|---|---|---|---|
| | %C | %H | %N |
| Calculated | 67.78 | 6.26 | 7.90 |
| Found | 67.48 | 6.39 | 7.72 |

### Example 4

### 2,2-Dimethyl-propionic acid 4-(1H-benzimidazol-2-yl)-2-chloro-phenyl ester

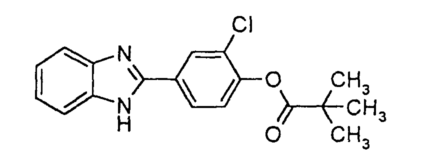

Initially, 13.5 mL of triethylamine were added dropwise to a stirred solution composed of 8.76 g (0.036 mol) of 2-(3-chloro-4-hydroxyphenyl)benzimidazole in 45 mL of anhydrous CH₂Cl₂, using external cooling with an ice-water bath, and next, 4.32 g (0.036 mol) of 2, 2-dimethylpropionyl chloride were added. Once the addition was completed, the resultant mixture was stirred at about 0°C for 30 minutes, and then, at room temperature for 4 hours. After that, 75 mL of ethyl ether were added to the mixture, the insoluble residue was filtered off, and the liquid was washed with H₂O (2 x 100 ml). Then, the organic phase was dried over anhydrous Na₂SO₄, the solvent was evaporated under reduced pressure, and the product was obtained as a solid with m.p. 206-8°C (recrystallized in methanol) with a yield of 74%, with 1/2 methanol molecule.

| **Quantitative Analysis .**Calculated for C₁₈H₁₇ClN₂O₂.1/2CH₄O | | | |
|---|---|---|---|
| | %C | %H | %N |
| Calculated | 64.44 | 5.55 | 8.12 |
| Found | 64.35 | 6.20 | 7.97 |

### Example 5

### 2,2-Dimethyl-propionic acid 4-(1H-benzoimidazol-2-yl)-2-nitro-phenyl ester

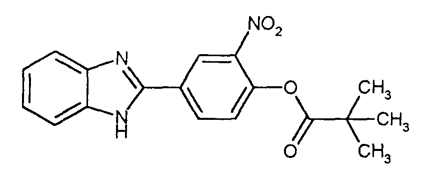

Initially, 12 mL of triethylamine were added dropwise to a stirred solution composed of 8 g (0.031 mol) of 2-(3-nitro-4-hydroxyphenyl)benzimidazole in 40 mL of anhydrous CH₂Cl₂, using external cooling with an ice-water bath. Then, 3.78 g (0.031 mol) of 2, 2-dimethylpropionyl chloride were added. Once the addition was completed, the resultant mixture was stirred at about O °C for 30 minutes, and next, at room temperature for 4 hours. After that, 70 mL of ethyl ether were added to the reaction mixture, the insoluble residue was filtered off and the liquid was washed with H₂O (2 x 100 ml). Then, the organic phase was dried over anhydrous Na₂SO₄, the solvent removed under reduced pressure, and the product was obtained as a solid with m.p. 185-7 °C (recrystallized in methanol) with a yield of 65%.

| **Quantitative Analysis**: Calculated for C₁₈H₁₇N₃O₄ | | | |
|---|---|---|---|
| | %C | %H | %N |
| Calculated | 63.71 | 5.05 | 12.38 |
| Found | 63.69 | 5.28 | 12.24 |

### Example 6

### 2,2-Dimethyl-propionic acid 4-(1H-benzimidazol-2-yl)-2-nitro-6-methoxy-phenyl ester

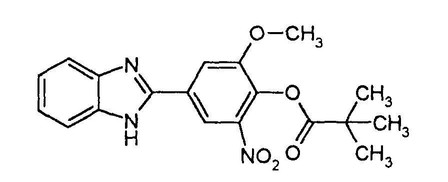

Initially, 13.5 mL of triethylamine were added dropwise to a stirred solution of 10 g (0.035 mol) of 2-(4-hydroxy-5-methoxy-3-nitrophenyl)benzimidazole in 45 mL of anhydrous CH₂Cl₂, using external cooling with an ice-water bath, and then, 4.23 g (0.035 mol) of 2, 2-dimethylpropionyl chloride. Once the addition was completed, the mixture was stirred at about 0°C for 30 minutes and next, at room temperature for 8 hours. Finally, 45 mL of ethyl ether were added to the mixture, the insoluble residue was filtered off and the remaining liquid was washed with H₂O (2 x 200 mL). The organic phase was dried over anhydrous Na₂SO₄, the solvent removed under reduced pressure, and the product was obtained as a solid with m.p. 190-2°C (recrystallized in ethyl acetate) with a yield of 50%.

| **Quantitative Analysis:**Calculated for C₁₉H₁₉N₃O₅ | | | |
|---|---|---|---|
| | %C | %H | %N |
| Calculated | 61.78 | 5.18 | 11.38 |
| Found | 62.02 | 5.51 | 11.04 |

### Example 7

### 2,2-Dimethyl-propionic acid 4-(5-Chloro-1H-benzimidazol-2-yl)phenyl ester

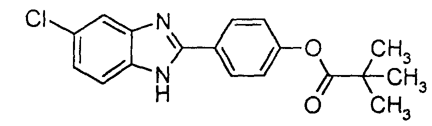

Initially, 13.5 mL of triethylamine were added dropwise to a stirred solution of 8.76 g (0,036 mol) of 2-(4-hydroxyphenyl)-5-chlorobenzimidazole in 45 mL of anhydrous CH₂Cl₂, using external cooling with an ice-water bath, and then, 4.32 g (0.036 mol) of 2, 2-dimethylpropionyl chloride were added. Once the addition was completed, the resultant mixture was stirred at about 0°C for 30 minutes and then, at room temperature for 8 hours. After such a time, 75 mL of ethyl eter were added to the mixture, the insoluble residue was filtered off, and the remaining liquid was washed with H₂O (2 x 100 ml). Then, the organic phase was dried over anhydrous Na₂SO₄, the solvent evaporated under reduced pressure, and the product was obtained as a solid with m.p. 247-9°C (recrystallized in ethanol) with a yield of 69%.

| **Quantitative Analysis :** Calculated for C₁₈H₁₇ClN₂O₂ | | | |
|---|---|---|---|
| | %C | %H | %N |
| Calculated | 65.75 | 5.21 | 8.52 |
| Found | 66.04 | 5.04 | 8.43 |

### Example 8

### 2,2-Dimethyl-propionic acid 4-(5-Chloro-1 H-benzimidazol-2-yl)-2-methoxy-phenyl ester

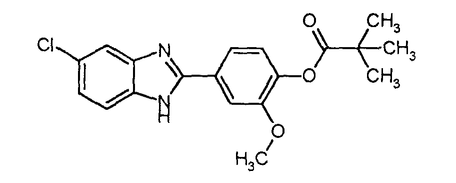

Initially, 15 mL of triethylamine were added dropwise to a stirred solution of 10 g (0.036 mol) of 2-(4-hydroxy-3-methoxyphenyl)-5-chlorobenzimidazole in 50 mL of anhydrous CH₂Cl₂, using external cooling with an ice-water bath, and then, 2.43 g (0.020 mol) of trimethylacetyl chloride. Once the addition was completed, the resultant mixture was stirred at about 0°C for 30 minutes and then, at room temperature for 4 hours. After such a time, 50 mL of ethyl ether were added to the reaction mixture, the insoluble residue was filtered and the liquid was washed with H₂O (2 x 125 ml). Then, the organic phase was dried over anhydrous Na₂SO₄, the solvent was evaporated under reduced pressure, and the product was obtained as a solid with m.p. = 197-9°C (recristallized in methanol) with a yield of 71%.

| **Quantitative Analysis:** Calculated for C₁₉H₁₉ClN₂O₃ | | | |
|---|---|---|---|
| | %C | %H | %N |
| Calculated | 63.60 | 5.34 | 7.81 |
| Found | 63.58 | 5.43 | 7.80 |

### Example 9

### 2,2-Dimethyl-propionic acid 4-(5,6-dimethyl-1H-benzimidazol-2-yl)phenyl ester

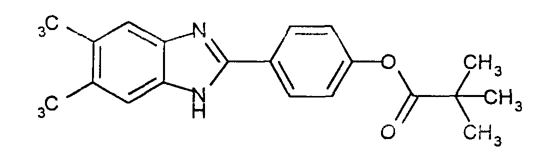

Initially, 13.5 mL of triethylamine were added dropwise to a stirred solution composed of 8.54 g (0.036 mol) of 2-(4-hydroxyphenyl)-5,6-dimethylbenzimidazole in 45 mL of anhydrous CH₂Cl₂ using external cooling with an ice-water bath, and next, 4.32 g (0.036 mol) of 2, 2-dimethylpropionyl chloride. Once the addition was completed, the resultant mixture was stirred at about 0°C for 30 minutes and next, at room temperature for 8 hours. After that, 75 mL of ethyl ether were added to the reaction mixture, the insoluble residue was filtered off and the liquid was washed with H₂O (2 x 100 ml). Then, the organic phase was dried over anhydrous Na₂SO₄, the solvent evaporated under reduced pressure and the product was obtained as a solid with m.p. 231-3 °C (recrystallized in ethanol/water) with a yield of 59%.

| **Quantitative Analysis:** Calculated for C₂₀H₂₂N₂O₂ | | | |
|---|---|---|---|
| | %C | %H | %N |
| Calculated | 74.51 | 6.88 | 8.69 |
| Found | 74.26 | 7.35 | 8.62 |

### Example 10

### 2,2-Dimethyl-propionic acid 4-(5-methyl-1H-benzimidazol-2-yl)phenyl ester

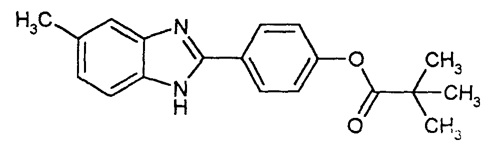

Initially, 16 mL of triethylamine were added dropwise top a stirred solution composed of 10 g (0.045 mol) of 2-(4-hydroxyphenyl)-5-methylbenzimidazole in 55 mL of anhydrous CH₂Cl₂, using external cooling with an ice-water bath, and next, 5.38 g (0.045 mol) of 2, 2-dimethylpropionyl chloride were added. Once the addition was completed, the mixture was stirred at about 0°C for 30 minutes and, then, at room temperature for 14 hours. Finally, 100 mL of ethyl ether were added to the mixture, the insoluble residue was filtered off and the liquid was washed with H₂O (2 x 125 mL). The organic phase were dried over anhydrous Na₂SO₄, the solvent removed under reduced pressure and the product was isolated as a solid with m.p. 235-7°C (recrystallized in ethyl acetate with a yield of 55 %),

| **Quantitative Analysis :** Calculated for C₁₉H₂₀N₂O₂ | | | |
|---|---|---|---|
| | %C | %H | %N |
| Calculated | 74.00 | 6.54 | 9.08 |
| Found | 74.32 | 6.61 | 9.19 |

### Example 11

### 2,2-Dimethyl-propionic acid 4-(5-methyl-1 H-benzimidazol-2-yl)-2-methoxy-phenyl ester

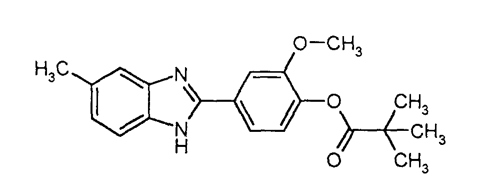

Initially, 15 mL of triethylamine were added dropwise to a stirred solution of 10 g (0.039 mol) of 2-(4-hydroxy-3-methoxyphenyl)-5-methylbenzimidazole in 50 mL of anhydrous CH₂Cl₂, using external cooling with an ice water bath. Then, 4.74 g (0.024 mol) of trimethylacetyl chloride were added. Once the addition was completed, teh mixture was stirred at about 0°C for 30 minutes and, next, at room temperature for 4 hours. At the end, 50 mL of ethyl ether were added to the mixture, the insoluble residue was filtered off, and the liquid was washed with H₂O (2 x 100 mL). The organic phase was dried over Na₂SO₄, the solvent evaporated under reduced pressure and the product obtained as a solid with m.p. 186-8°C (recrystallized in n-hexane/ethyl acetate 1:1) with a yield of 59%.

| **Quantitative Analysis:** Calculated for C₂₀H₂₂N₂O₃ | | | |
|---|---|---|---|
| | %C | %H | %N |
| Calculated | 70.99 | 6.55 | 8.28 |
| Found | 70.98 | 6.61 | 8.02 |

### Example 12

### 2,2-Dimethyl-propionic acid 4-(5,6-dimethyl-1 H-benzimidazol-2-yl)-2-methoxyphenyl ester

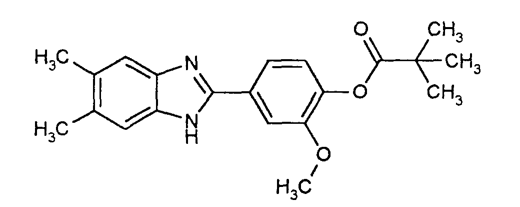

Initially, 14 mL of triethylamine were added dropwise to a stirred solution of 10 g (0.037 mol) of 2-(4-hydroxy-3-methoxyphenyl)-5,6-dimethylbenzimidazole in 50 mL of anhydrous CH₂Cl₂, using external cooling with an ice-water bath, and then, 4.49 g (0.037 mol) of 2, 2-dimethylpropionyl chloride were added. Once the addition was completed, the mixture was stirred at about 0°C for 30 minutes, and then, at room temperature for 4 hours. After such a time, 50 mL of ethyl ether were added to the mixture, the insoluble residue was filtered off, and the liquid was washed with H₂O (2 x 100 ml). Then, the organic phase was dried over anhydrous Na₂SO₄, the solvent evaporated under reduced pressure, and the product was obtained as a solid with m.p. = 177-9°C (recrystallized in n-hexane/ethyl acetate 1:1) with a yield of 70%.

| **Quantitative Analysis:** Calculated for C₂₁H₂₄N₂O₃ | | | |
|---|---|---|---|
| | %C | %H | %N |
| Calculated | 71.57 | 6.86 | 7.95 |
| Found | 71.03 | 7.10 | 7.69 |

### Example 13

### 2,2-Dimethyl-propionic acid 4-(5-nitro-1 H-benzimidazol-2-yl)phenyl ester

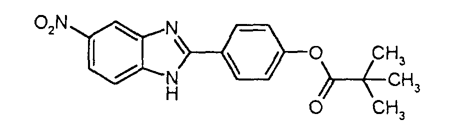

Initially, 0,5 g (0.004 mol) of 4-dimethylaminopyridine were added dropwise to a stirred solution of 10.21 g (0.04 mol) of 2-(4-hydroxy)-5-nitrobenzimidazole in 60 mL of anhydrous CHCl₃, using external cooling with an ice-water bath, and next, 7.45 g (0.04 mol) of 2, 2-dimethylpropionyl anhydride. Once the addition was completed, the mixture was stirred at room temperature for 12 hours. After such a time, about 40 ml of the solvent were evaporated under reduced pressure, and the resultant mixture was cooled at -10°C overnight. Then, the crystallized product was separated by filtration, yielding a solid with m.p. 198-200°C (recrystallized in ethyl acetate) with a yield of 33%.

| **Quantitative Analysis:** Calculated for C₁₈H₁₇N₃O₄ | | | |
|---|---|---|---|
| | %C | %H | %N |
| Calculated | 63.71 | 5.05 | 12.38 |
| Found | 63.19 | 5.23 | 12.20 |

### Example 14

### 2,2-Dimethyl-propionic acid 4-(5-nitro-1 H-benzimidazol-2-yl)-6-methoxy-2-nitrophenyl ester

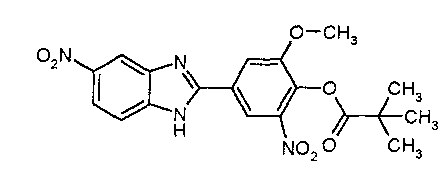

Initially, 11 mL of triethylamine were added dropwise to a stirred solution of 10 g (0.03 mol) of 2- (4-hydroxy-5-methoxy-3-nitrophenyl)-5-nitrobenzimidazole in 38 mL of anhydrous CH₂Cl₂, using external cooling with an ice-water bath, and next, 3.65 g (0.03 mol) of 2, 2-dimethylpropionyl chloride were added. Once the addition was completed, the resultant mixture was stirred at about 0°C for 30 minutes and then, at room temperature for 8 hours. Then, 40 mL of ethyl ether were added to the mixture, the insoluble residue was filtered off and the liquid was washed with H₂O (2 x 175 mL). Finally, the organic phase was dried over anhydrous Na₂SO₄, the solvent evaporated under reduced pressure, and the product was isolated as a solid with m.p. 243-5°C (recrystallized in n-hexane/ethyl acetate 1:1) with a yield of 49%.

| **Quantitative Analysis:** Calculated for C₁₉H₁₈N₄O₇ | | | |
|---|---|---|---|
| | %C | %H | %N |
| Calculated | 55.07 | 4.38 | 13.52 |
| Found | 55.08 | 4.39 | 13.24 |

### Example 15

### 2,2-Dimethylpropionic acid 4-[1-(2-dimethylaminoethyl)-1H-benzimidazol-2-yl] phenyl ester. (MAH-1)

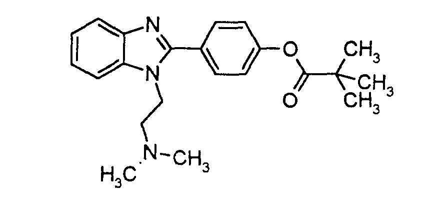

To a stirred solution of the 4-[1-(2-dimethylamino-ethyl)-1H-benzimidazol-2-yl]phenol (0.5 g, 1.78 mmol) and NaOH ( 0.36 g , 8.89 mmol) in dry CH₂Cl₂ (50 mL) at room temperature was added pivaloyl chloride (0.32 g, 2.67 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel ,eluting with EtOAc/acetone (5/1) to give a white solid, which was recrystallized from diethyl ether, and had a melting point of 107-109 °C. Yield: 86%

| **Quantitative Analysis:** Calculated for C₂₂H₂₇N₃O₂ (365.48 g/mol) | | | |
|---|---|---|---|
| | %C | %H | %N |
| Calculated | 72.30 | 7.45 | 11.50 |
| Found | 72.49 | 7.50 | 11.20 |

### Example 16

### 2,2-Dimethylpropionic acid 2-Bromo-4-[1-(2-dimethylaminoethyl)-1H-benzimidazol-2-yl]phenyl ester. (MAH-4)

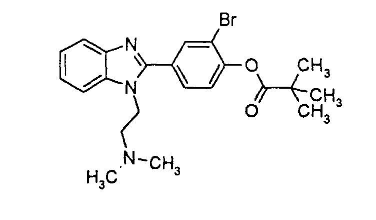

To a stirred solution of the 2-bromo-4-[1-(2-dimethylamino-ethyl)-1H-benzimidazol-2-yl]phenol (0.65 g, 1.78 mmol) and NaOH ( 0.36 g , 8.89 mmol) in dry CH₂Cl₂ (50 mL) at room temperature was added pivaloyl chloride (0.32 g, 2.67 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel , using as eluent EtOAc/acetone (5/1) to give a white solid, which was recrystallized from hexane, giving a melting point of 117-118 °C. Yield: 75%.

| **Quantitative Analysis :** Calculated for C₂₃H₂₉N₃O₂ (379.50 g/mol): | | | |
|---|---|---|---|
| | %C | %H | %N |
| Calculated | 59.46 | 5.90 | 9.46 |
| Found | 59.08 | 5.78 | 9.86 |

### Example 17

### 2,2-Dimethylpropionic acid 4-[1 -(2-dimethylaminopropyl)-1 H-benzimidazol-2-yl]phenyl ester, dihydrogen oxalate. (MAH-2)

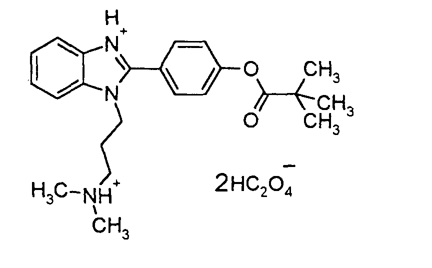

To a stirred solution of the 4-[1-(2-dimethylamino-propyl)-1 H-benzimidazol-2-yl]phenol (0.52 g, 1.78 mmol) and NaOH ( 0.36 g, 8.89 mmol) in dry CH₂Cl₂ (50 mL) at room temperature was added pivaloyl chloride (0.32 g, 2.67 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel , eluting with acetone to give a colourless oil, which was isolated as oxalate. The salt was recristallyzed from EtOH, giving a melting point of 157-159 °C. Yield: 54%

| **Quantitative Analysis :** Calculated for C₂₇H₃₃N₃O₁₀.H₂O (577.59 g/mol): | | | |
|---|---|---|---|
| | %C | %H | %N |
| Calculated | 56.14 | 6.11 | 7.27 |
| Found | 56.50 | 6.02 | 7.25 |

### Example 18

### 2,2-Dimethylpropionic acid 4-[1-(2-diisopropylaminoethyl)-1 H-benzimidazol-2-yl]phenyl ester. (MAH-3)

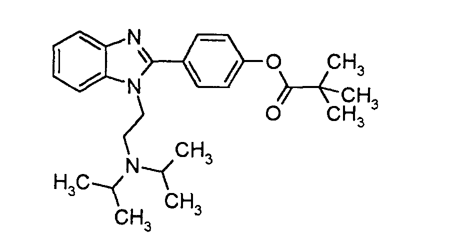

To a stirred solution of the 4-[1-(2-diisopropylamino-ethyl)-1H-benzimidazol-2-yl]phenol (0.6 g, 1.78 mmol) and NaOH ( 0.36 g, 8.89 mmol) in dry CH₂Cl₂ (50 mL) at room temperature was added pivaloyl chloride (0.32 g, 2.67 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel, using as eluent hexane/EtOAc (7/3) to give a white solid, which was recrystallized from hexane, giving a melting point of 143-144 °C. Yield: 70%.

| **Quantitative Analysis :** Calculated for C₂₆H₃₅N₃O₂ (421.58 g/mol) | | | |
|---|---|---|---|
| | %C | %H | %N |
| Calculated | 74.07 | 8.37 | 9.97 |
| Found | 73.67 | 8.28 | 10.31 |

### Example 19

### 2,2-Dimethylpropionic acid 4-[5,6-dichloro-1-(2-dimethylaminoethyl) 1H-benzimidazol-2-yl] phenyl ester. (MAH-7)

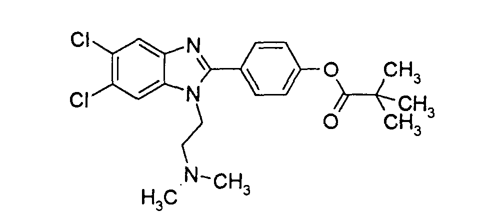

To a stirred solution of the 4-[5,6-dichloro-3-(2-dimethylamino-ethyl)-1H-benzimidazol-2-yl]phenol (1g, 2.8 mmol) and NaOH ( 0.57 g, 14.2 mmol) in dry CH₂Cl₂ (50 mL) at room temperature was added pivaloyl chloride (0.67 g, 5.67 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel , eluting with EtOAc to give a white solid, which was recrystallized from hexane, giving a melting point of 140-141 °C. Yield: 59%.

| **Quantitative Analysis :** Calculated for C₂₂H₂₅Cl₂N₃O₂ (434.37 g/mol) | | | |
|---|---|---|---|
| | %C | %H | %N |
| Calculated | 60.83 | 5.80 | 9.67 |
| Found | 60.55 | 6.14 | 9.63 |

### Example 20

### 2,2-Dimethylpropionic acid 4-[5,6-dimethyl-3-(2-piperidin-1-yl-ethyl)-1H-benzimidazol-2-yl] phenyl ester. (MAH-8)

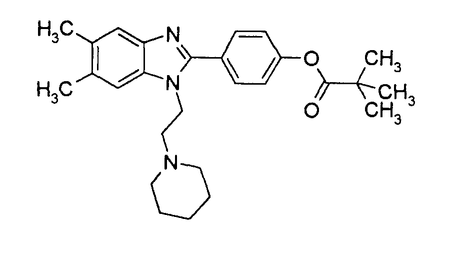

To a stirred solution of the 4-[5,6-dimethyl-3-(2-dimethylamino-ethyl)-1H-benzimidazol-2-yl]phenol (1.2 g, 3.4 mmol) and NaOH (1.17 g, 17.2 mmol) in dry CH₂Cl₂ (100 mL) at room temperature was added pivaloyl chloride (0.82 g, 6.86 mmol). The mixture was stirred for 5 h and then H₂O (150 mL ) was added. The organic layer was separeted and the aqueous layer was extracted with CH₂Cl₂ (2x25 ml). The combined organic layers were dried over Na₂SO₄ and concentrated under reduced pressure. The residue was purified by column chromatography on silica gel , eluting with EtOAc to give a white solid, which was recrystallized from hexane, giving a melting point of 144-145 °C. Yield: 74%.

| **Quantitative Analysis :** Calculated for C₂₇H₃₅N₃O₂ (433.59 g/mol): | | | |
|---|---|---|---|
| | %C | %H | %N |
| Calculated | 74.79 | 8.14 | 9.69 |
| Found | 74.86 | 8.43 | 9.48 |

### Example 21

### 2,2-Dimethylpropionic acid 2-fluoro-4-[1-(2-piperidin-1-yl ethyl)-1 H-benzimidazol-2-yl] phenyl ester. (MAH-10)

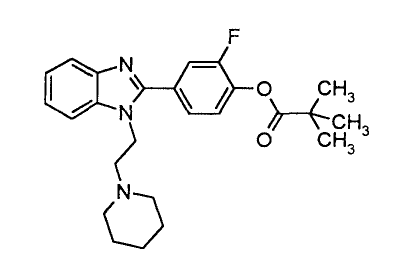

To a stirred solution of the 4-[3-(2-dimethylamino-ethyl)-1H-benzimidazol-2-yl]-2-fluorophenol (1.4 g, 4.15 mmol) and NaOH ( 0.82 g, 20.7 mmol) in dry CH₂Cl₂ (100 mL) at room temperature was added pivaloyl chloride (1.0 g, 8.29 mmol). The mixture was stirred for 5 h and then H₂O (150 mL ) was added. The organic layer was separated and the aqueous layer was extracted with CH₂Cl₂ (2x25 ml). The combined organic layers were dried over Na₂SO₄ and concentrated under reduce pressure. The residue was purified by column chromatography on silica gel, eluting with EtOAc to give a white solid, which was recrystallized from hexane, giving a melting point of 147-148 °C. Yield: 68%.

| **Quantitative Analysis :** Calculated for C₂₅H₃₀FN₃O₂ (423.53 g/mol): | | | |
|---|---|---|---|
| | %C | %H | %N |
| Calculated | 70.90 | 7.14 | 9.92 |
| Found | 70.76 | 7.18 | 9.98 |

### Example 22

### 2,2-Dimethyl-propionic acid 2-(1H-benzimidazol-2-yl)phenyl ester

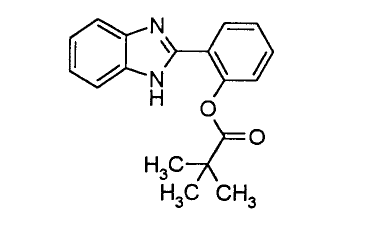

Initially, 18 mL of triethylamine were added dropwise to a stirred solution composed of 10 g (0.048 mol) of 2-(2-hydroxyphenyl)benzimidazole in 60 mL of anhydrous CH₂Cl₂, using external cooling with an ice-water bath. Then, 5.73 g (0.048 mol) of 2, 2-dimethylpropionyl chloride were added. Once the addition was completed, the resultant mixture was stirred at about O°C for 30 minutes and then, at room temperature for 8 hours. At the end, 100 mL of ethyl ether were added to the reaction mixture, the insoluble residue was filtered off and the liquid was washed with H₂O (2 x 150 ml). Then, the organic phase was dried over anhydrous Na₂SO₄. Finally, after evaporating the solvent under reduced pressure, the product was isolated as a solid with m.p. 147-9°C (recrystallized in ethyl acetate) with a yield of 73%.

| **Quantitative Analysis:** Calculated for C₁₈H₁₈N₂O₂ | | | |
|---|---|---|---|
| | %C | %H | %N |
| Calculated | 73.45 | 6.16 | 9.52 |
| Found | 73.72 | 6.30 | 9.44 |

### Example 23

### 2,2-Dimethyl-propionic acid 2-(1H-benzimidazol-2-yl)-4-chloro-phenyl ester

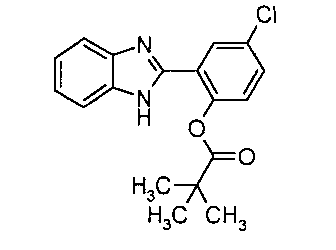

Initially, 376.2 g (4.76 mol) of pyridine were added dropwise to a stirred solution of 116.4 g (0.48 mol) of 2-(3-chloro-6-hydroxyphenyl)benzimidazole in 750 mL of anhydrous acetone, using external cooling with an ice-water bath, and then, 573.5 g (4.76 mol) of 2, 2-dimethylpropionyl chloride. Once the addition was completed, the resultant mixture was stirred at room temperature for 6 hours. At the end, the reaction mixture was poured into water-ice (1.5 L), and the resulting solution was made alkaline with K₂CO₃. Finally, the precipitate was filtered and washed with H₂O, until liquids appear neutral. In this way, the product was obtained as a solid with m.p.189-91°C (recrystallized in ethyl acetate) with a yield of 71%

| **Quantitative Analysis:** Calculated for C₁₈H₁₇ClN₂O₂ | | | |
|---|---|---|---|
| | %C | %H | %N |
| Calculated | 65.75 | 5.21 | 8.52 |
| Found | 65.71 | 5.28 | 8.31 |

### Example 24

### 2,2-Dimethyl-propionic acid 2-(1H-benzimidazol-2-yl)-5-chloro-phenyl ester

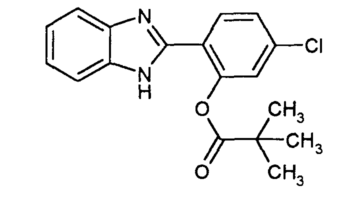

Initially, 14 mL of triethylamine were added dropwise to a stirred solution of 10 g (0.041 mol) of 2-(4-chloro-2-hydroxyphenyl)benzimidazole in 47 mL of anhydrous CH₂Cl₂, using external cooling with an ice-water bath, and next, 4.93 g (0.041 mol) of 2, 2-dimethylpropionyl chloride were added. Once the addition was completed, the resulting mixture was stirred at about 0°C for 30 minutes and, then, at room temperature for 4 hours. After such a time, 45 mL of ethyl ether were added to the mixture, the insoluble residue was filtered off, and the liquid was washed with H₂O (2 x 100 mL). Then, the organic phase was dried over Na₂SO₄, the solvent evaporated under reduced pressure and the product was obtained as a solid with m.p. 147-9°C (recrystallized in diisopropyl ether) with a yield of 56 %.

| **Quantitative Analysis :** Calculated for C₁₈H₁₇, ClN₂O₂ | | | |
|---|---|---|---|
| | %C | %H | %N |
| Calculated | 65.75 | 5.21 | 8.52 |
| Found | 65.84 | 5.29 | 8.51 |

### Example 25

### 2,2-Dimethyl-propionic acid 2-(1H-benzimidazol-2-yl)-4,6-dichloro-phenyl ester

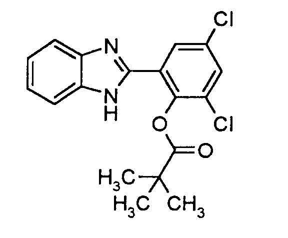

Initially, 13.5 mL of triethylamine were added dropwise to a stirred solution of 10 g (0.036 mol) of 2-(3,5-dichloro-2-hydroxyphenyl)benzimidazole in 45 mL of anhydrous CH₂Cl₂, using external cooling with an ice-water bath. Then, 4.32 g (0.036 mol) of 2, 2-dimethylpropionyl chloride were added. Once the adition was completed, the mixture was stirred at O°C for 30 minutes and then, at room temperature for 11 hours more. After that, 75 mL of ethyl ether were added to the reaction mixture, the insoluble residue was filtered off, and the liquid was washed with H₂O (2 x 100 ml). Then, the organic phase was dried over anhydrous Na₂SO₄. Finally, after evaporating the solvent under reduced pressure, the product was isolated as a solid with m.p. 220-2°C (recrystallized in ethanol) with a yield of 67 %.

| **Quantitative Analysis:** Calculated for C₁₈H₁₆Cl₂N₂O₂ | | | |
|---|---|---|---|
| | %C | %H | %N |
| Calculated | 59.52 | 4.44 | 7.71 |
| Found | 59.86 | 4.67 | 7.98 |

### Example 26

### 2,2-Dimethyl-propionic acid 2-(1H-benzimidazol-2-yl)-6-methoxy-phenyl ester

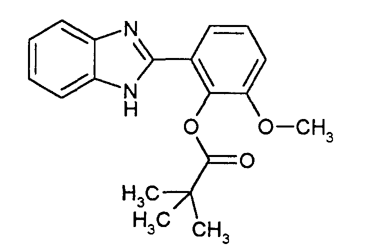

Initially, 15 mL of triethylamine were added dropwise to a stirred solution composed of 10 g (0.042 mol) of 2-(2-hydroxy-3-methoxyphenyl)benzimidazole in 51 mL of anhydrous CH₂Cl₂, using external cooling with an ice-water bath, and next, 5.02 g (0.042 mol) of 2, 2-dimethylpropionyl chloride. Once the addition was completed, the resultant mixture was stirred at about 0°C for 30 minutes and next, at room temperature for 4 hours. Then, 45 mL of ethyl ether were added to the mixture, the insoluble residue was filtered off and the liquid was washed with H₂O (2 x 100 mL) . Then, the organic phase was dried over anhydrous Na₂SO₄, the solvent evaporated under reduced pressure, and the product was obtained as a solid with m.p. 158- 60°C (recrystallized in ethyl acetate) with a yield of 82%.

| **Quantitative Analysis:** Calculated for C₁₉H₂₀N₂O₃ | | | |
|---|---|---|---|
| | %C | %H | %N |
| Calculated | 70.35 | 6.21 | 8.64 |
| Found | 70.74 | 6.28 | 8.62 |

### Example 27

### 2,2-Dimethyl-propionic acid 2-(5-chloro-1H-benzimidazol-2-yl)phenyl ester

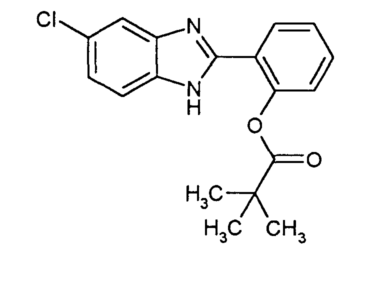

Initially, 12 mL of triethylamine were added dropwise to a stirred solution composed of 8 g (0.033 mol) of 2-(2-hydroxyphenyl)-5-chlorobenzimidazole in 40 mL of anhydrous CH₂Cl₂ using external cooling with an ice-water bath, and next, 3.94 g (0.033 mol) of 2, 2-dimethylpropionyl chloride. Once the addition was completed, the resultant mixture was stirred at about O °C for 30 minutes and then, at room temperature for 8 hours. Finally, 50 mL of ethyl ether were added to the mixture, the insoluble residue was filtered off and the liquid was washed with H₂O (2 x 100 mL). Then, the organic phase was dried over anhydrous Na₂SO₄, the solvent removed under reduced pressure, and the product was obtained as a solid with m.p. 178-80 °C (recrystallized in ethyl acetate) with a yield of 49%.

| **Quantitative Analysis:** Calculated for C₁₈H₁₇ClN₂O₂ | | | |
|---|---|---|---|
| | %C | %H | %N |
| Calculated | 65.75 | 5.21 | 8.52 |
| Found | 65.52 | 5.42 | 8.46 |

### Example 28

### 2,2-Dimethyl-propionic acid 2-(-5-chloro-1 H-benzimidazol-2-yl)-5-diethylaminophenyl ester

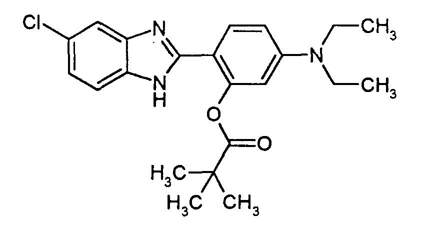

Initially, 6 mL of triethylamine were added dropwise to a stirred solution composed of 4.5 g (0.014 mol) of 2-(2-hydroxy-4-diethylamino)-5-chlorobenzimidazole in 30 mL of anhydrous CH₂Cl₂, using external cooling with an ice-water bath, and next, 1.89 g (0.016 mol) of 2, 2-dimethylpropionyl chloride were added. Once the addition was completed, the mixture were stirred at about 0°C for 30 minutes and then, at room temperature for 4 hours. Then, 20 mL of ethyl ether were added to the reaction mixture, the insoluble residue was filtered off and the filtered liquid was washed with H₂O (2 x 50 mL). The organic phase was dried over anhydrous Na₂SO₄, the solvent removed under reduced pressure, and the product was obtained as a solid with m.p. 194-6°C (recrystallized in ethyl acetate) with a yield of 66%.

| **Quantitative Analysis:** Calculated for C₂₂H₂₆ClN₃O₂ | | | |
|---|---|---|---|
| | %C | %H | %N |
| Calculated | 66.07 | 6.55 | 10.51 |
| Found | 66.12 | 6.67 | 10.39 |

### Example 29

### 2,2-Dimethyl-propionic acid 2-(5,6-dimethyl-1 H-benzimidazol-2-yl)phenyl ester

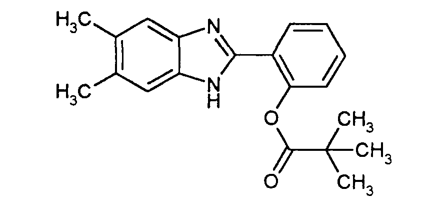

Initially, 13.5 mL of triethylamine were added dropwise to a stirred solution of 8.71 g (0.037 mol) of 2-(2-hydroxyphenyl)-5,6-dimethylbenzimidazole in 45 mL of anhydrous CH₂Cl₂, using external cooling with an ice-water bath, and next, 4.4 g (0.037 mol) of 2, 2-dimethylpropionyl chloride were added. Once the addition was completed, the resulting mixture was stirred for about 0°C for 30 minutes, and then, at room temperature for 5 hours. After that, 75 mL of ethyl ether were added to the mixture, the insoluble residue was filtered off and the liquid was washed with H₂O (2 x 200 mL). The organic phase was dried over anhydrous Na₂SO₄, the solvent evaporated under reduced pressure, and the product was isolated as a solid with m.p. 132-4°C (recrystallized in diisopropyl ether) with a yield of 65 %.

| **Quantitative Analysis :** Calculated for C₂₀H₂₂N₂O₂ | | | |
|---|---|---|---|
| | %C | %H | %N |
| Calculated | 74.51 | 6.88 | 8.69 |
| Found | 74.81 | 7.24 | 8.69 |

### Example 30

### 2,2-Dimethyl-propionic acid 2-(5-methyl-1 H-benzimidazol-2-yl)-4-chloro-phenyl ester

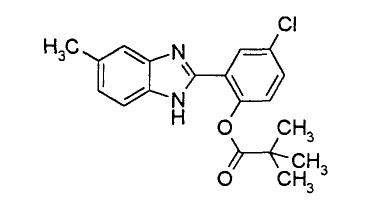

Initially, 15 mL of triethylamine were added dropwise to a stirred solution of 7 g (0.027 mol) of 2-(3-chloro-6-hydroxyphenyl)-5-methylbenzimidazole in 50 mL of anhydrous CH₂Cl₂, using external cooling with an ice-water bath, and then, 4.49 g (0.037 mol) of 2, 2-dimethylpropionyl chloride were added. Once the addition was completed, the mixture was stirred at about 0°C for 30 minutes and next, at room temperature for 4 hours. After such a time, 50 mL of ethyl ether were added to the mixture, the insoluble residue was filtered and the remaining liquid was washed with H₂O (2 x 100 mL). Then, the organic phase was dried over anhydrous Na₂SO₄, the solvent was evaporated under reduced pressure, and the product was obtained as a solid with m.p. 162-4°C (recrystallized in n-hexane/ethyl acetate 1:1) with a yield of 59%.

| **Quantitative Analysis:** Calculated for C₁₉H₁₉ClN₂O₂ | | | |
|---|---|---|---|
| | % C | % H | % N |
| Calculated | 66.57 | 5.59 | 8.17 |
| Found | 66.52 | 5.67 | 8.13 |

### Example 31

### 2,2-Dimethyl-propionic acid 2-(5,6-dimethyl-1 H-benzimidazol-2-yl)-diethylaminophenyl ester

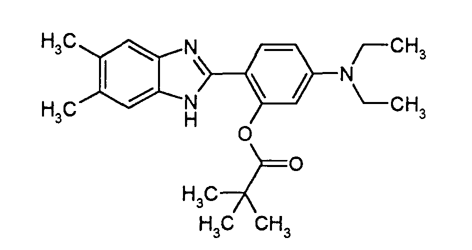

Initially, 7.5 mL of triethylamine were added dropwise to a stirred solution of 4.15 g (0.013 mol) of 2-(2-hydroxy-4-diethylaminophenyl)-5,6-dimethylbenzimidazole in 25 mL of anhydrous CH₂Cl₂, using external cooling with an ice-water bath, and then, 2.43 g (0.020 mol) of 2, 2-dimethylpropionyl chloride. Once the addition was completed, the mixture was stirred at about 0°C for 30 minutes and then, at room temperature for 4 hours. After such a time, 25 mL of ethyl ether were added to the mixture, the insoluble residue was filtered off, and the liquid was washed with H₂O (2 x 50 ml). Then, the organic phase was dried over anhydrous Na₂SO₄, the solvent was evaporated under reduced pressure, and the product was obtained as a solid with m.p. = 160-2°C (recrystallized in ethyl acetate) with a yield of 61 %.

| **Quantitative Analysis:** Calculated for C₂₄H₃₁N₃O₂ | | | |
|---|---|---|---|
| | %C | %H | %N |
| Calculated | 73.25 | 7.94 | 10.68 |
| Found | 73.24 | 7.63 | 11.21 |

### Example 32

### 2,2-Dimethyl-propionic acid 2-(5-nitro-1 H-benzimidazol-2-yl)-phenyl ester

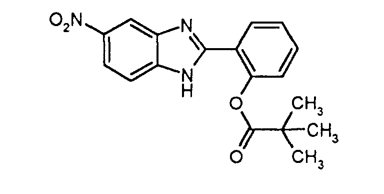

Initially, 14 mL of triethylamine were added dropwise to a stirred solution of 10 g (0.039 mol) of 2-(2-hydroxyphenyl)-5-nitrobenzimidazole in 50 mL of anhydrous CH₂Cl₂, using external cooling with an ice-water bath, and then, 7.09 g (0.059 mol) of 2, 2-dimethylpropionyl chloride were added. Once the addition was completed, the mixture was stirred at about O°C for 30 minutes, and then, at room temperature for 4 hours. After such a time, 50 mL of ethyl ether were added to the mixture, the insoluble residue was filtered off, and the liquid was washed with H₂O (2 x 100 ml). Then, the organic phase was dried over anhydrous Na₂SO₄, the solvent evaporated under reduced pressure, and the product was obtained as a solid with m.p. = 156-8°C (recrystallized in n-hexane/ethyl acetate 1:1) with a yield of 49%.

| **Quantitative Analysis:** Calculated for C₁₈H₁₇N₃O₄ | | | |
|---|---|---|---|
| | %C | %H | %N |
| Calculated | 63.71 | 5.05 | 12.38 |
| Found | 63.81 | 5.21 | 12.55 |

### Example 33

### 2,2-Dimethyl-propionic acid 2-(5-nitro-1 H-benzimidazol-2-yl)-4-chloro-phenyl ester

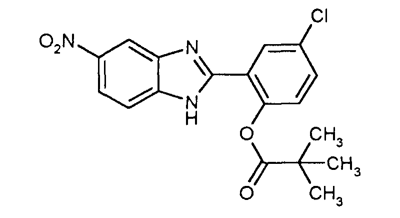

Initially, 14 mL of triethylamine were added dropwise to a stirred solution of 11 g (0.038 mol) of 2-(3-chloro-6-hydroxyphenyl)-5-nitrobenzimidazole in 47 mL of anhydrous CH₂Cl₂, using external cooling with an ice-water bath, and then, 4.6 g (0.038 mol) of 2, 2-dimethylpropionyl chloride. Once the addition was completed, the mixture was stirred at about O°C for 30 minutes and next, at room temperature for 4 hours. Then, 75 mL of ethyl ether were added to the reaction mixture, the insoluble residue was filtered off and the remaining liquid was washed with H₂O (2 x 100 mL). Then, the organic phase was dried over anhydrous Na₂SO₄, the solvent was removed under reduced pressure, and the product was isolated as a solid with m.p. 248-50 °C (recrystallized in ethyl acetate) with a yield of 71%.

| **Quantitative Analysis:** Calculated for C₁₈H₁₆ClN₃O₄ | | | |
|---|---|---|---|
| | % C | % H | % N |
| Calculated | 57.84 | 4.31 | 11.24 |
| Found | 57.87 | 4.35 | 11.08 |

### Example 34

### 2,2-Dimethyl-propionic acid 2-(5-nitro-1 H-benzimidazol-2-yl)-6-methyl-phenyl ester

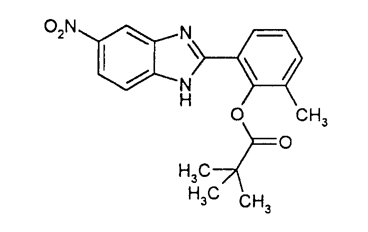

Initially, 9 mL of triethylamine were added dropwise to a stirred solution of 6.5 g (0.024 mol) of 2-(2-hydroxy-3-methyl)-5-nitrobenzimidazole in 30 mL of anhydrous CH₂Cl₂, using external cooling with an ice-water bath, and next, 2.91 g (0.024 mol) of 2, 2-dimethylpropionyl chloride. Once the addition was completed, the resultant mixture was stirred at about 0°C for 30 minutes and then, at room temperature for 8 hours. At the end, 30 mL of ethyl ether were added to the mixture, the insoluble residue was filtered off and the remaining liquid was washed with H₂O (2 x 100 mL) . Then, the organic phase was dried over anhydrous Na₂SO₄, the solvent removed under reduced pressure, and the product isolated as a solid with m.p. 198-200°C (recrystallized in ethyl acetate) with a yield of 35%.

| **Quantitative Analysis:** Calculated for C₁₉H₁₉N₃O₄ | | | |
|---|---|---|---|
| | %C | %H | %N |
| Calculated | 64.58 | 5.42 | 11.89 |
| Found | 64.76 | 5.46 | 11.86 |

### Example 35

### 2,2-Dimethyl-propionic acid 5-(1H-benzimidazol-2-yl)-phenyl ester

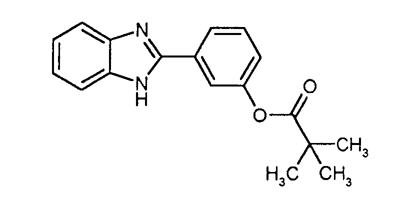

Initially, 17.5 mL of triethylamine were added dropwise to a stirred solution of 10 g (0.048 mol) of 2-(3-hydroxyphenyl)benzimidazole in 60 mL of anhydrous CH₂Cl₂, using external cooling with an ice-water bath, and then, 5.74 g (0.048 mol) of 2, 2-dimethylpropionyl chloride were added. Once the addition was completed, the mixture was stirred at about 0°C for 30 minutes and next, at room temperature for 14 hours. At the end, 100 mL of ethyl ether were added to the reaction mixture, the insoluble residue was filtered off and the liquid was washed with H₂O (2 x 125 mL). Then, the organic phase was dried over anhydrous Na₂SO₄, the solvent evaporated under reduced pressure, and the product was isolated as a solid with m.p. 243-5°C (recrystallized in ethyl acetate) with a yield of 41%.

| **Quantitative Analysis:** Calculated for C₁₈H₁₈N₂O₂ | | | |
|---|---|---|---|
| | %C | %H | %N |
| Calculated | 73.45 | 6.16 | 9.52 |
| Found | 73.80 | 6.51 | 9.39 |

### Example 36

### 2,2-Dimethyl-propionic acid 3-(1H-benzimidazol-2-yl)-6-methoxy-phenyl ester

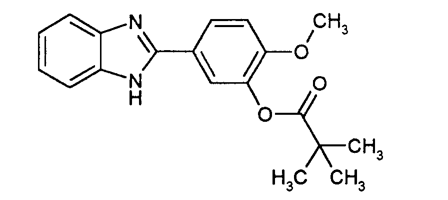

Initially, 15 mL of triethylamine were added dropwise to a stirred solution composed of 10 g (0.042 mol) of 2-(3-hydroxy-4-methoxyphenyl)benzimidazole in 50 mL of anhydrous CH₂Cl₂, using external cooling with an ice-water bath, and next, 5.02 g (0.042 mol) of 2, 2-dimethylpropionyl chloride. Once the addition was completed, the resultant mixture was stirred at about O°C for 30 minutes and next, at room temperature for 4 hours. Then, 75 mL of ethyl ether were added to the mixture, the insoluble residue was filtered off and the liquid was washed with H₂O (2 x 100 mL). Finally, the organic phase was dried over anhydrous Na₂SO₄, the solvent evaporated under reduced pressure, and the product was obtained as a solid with m.p. 202-4°C (recrystallized in ethyl acetate) with a yield of 88%.

| **Quantitative Analysis:** Calculated for C₁₉H₂₀N₂O₃ | | | |
|---|---|---|---|
| | % C | % H | % N |
| Calculated | 70.35 | 6.21 | 8.64 |
| Found | 70.28 | 6.38 | 8.29 |

### Example 37

### 2,2-Dimethyl-propionic acid 3-(1H-benzimidazol-2-yl)-4-nitro-phenyl ester

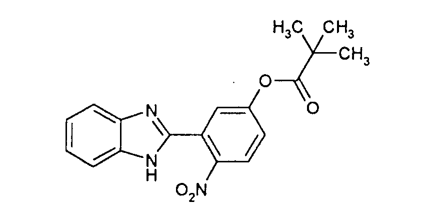

Initially, 14 mL of triethylamine were added dropwise to a stirred solution composed of 10 g (0.039 mol) of 2-(5-hydroxy-2-nitro)benzimidazole in 50 mL of anhydrous CH₂Cl₂, using external cooling with an ice-water bath, and next, 4.72 g (0.039 mol) of 2, 2-dimethylpropionyl chloride. Once the addition was completed, the mixture was stirred at about 0°C for 30 minutes and next, at room temperature for 4 hours. Finally, 100 mL of ethyl ether were added to the reaction mixture, the insoluble residue was filtered off and the liquid was washed with H₂O (2 x 200 ml). The organic phase was dried over anhydrous Na₂SO₄, the solvent evaporated under reduced pressure, and the product was isolated as a solid with m.p. 163-5°C (recrystallized in ethyl acetate) with a yield of 89%.

| **Quantitative Analysis:** Calculated for C₁₈H₁₇N₃O₄ | | | |
|---|---|---|---|
| | % C | % H | % N |
| Calculated | 63.71 | 5.05 | 12.38 |
| Found | 63.91 | 5.03 | 12.36 |

### Example 38

### 2,2-Dimethyl-propionic acid 3-(5-chloro-1 H-benzimidazol-2-yl)phenyl ester

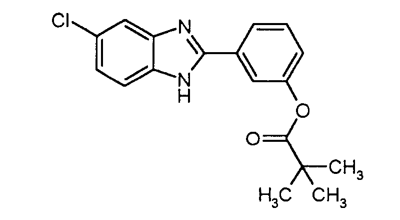

Initially, 13,5 mL of triethylamine were added dropwise to a stirred solution of 6 g (0.025 mol) of 2-(3-hydroxyphenyl)-5-chlorobenzimidazole in 45 mL of anhydrous CH₂Cl₂, using external cooling with an ice-water bath, and then, 4.44 g (0.037 mol) of 2, 2-dimethylpropionyl chloride were added. Once the addition was completed, the mixture was stirred at about O°C for 30 minutes, and then, at room temperature for 4 hours. After such a time, 45 mL of ethyl ether were added to the mixture, the insoluble residue was filtered off, and the liquid was washed with H₂O (2 x 100 ml). Then, the organic phase was dried over anhydrous Na₂SO₄, the solvent evaporated under reduced pressure, and the product was obtained as a solid with m.p. = 185-7°C (recrystallized in ethyl acetate) with a yield of 32%.

| **Quantitative Analysis:** Calculated for C₁₈H₁₇N₂O₂ | | | |
|---|---|---|---|
| | % C | % H | % N |
| Calculated | 65.75 | 5.21 | 8.52 |
| Found | 65.58 | 5.07 | 8.44 |

### Example 39

### 2,2-Dimethyl-propionic acid 3-(5,6-dimethyl-1H-benzimidazol-2-yl)phenyl ester

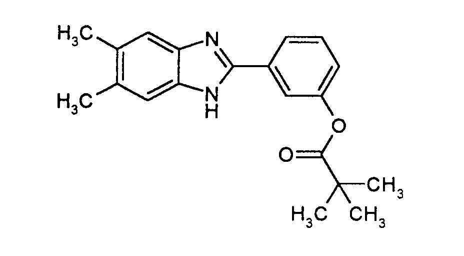

Initially, 13.5 mL of triethylamine were added dropwise to a stirred solution composed of 8.71 g (0.037 mol) of 2-(3-hydroxyphenyl)-5,6-dimethylbenzimidazole in 45 mL of anhydrous CH₂Cl₂, using external cooling with an ice-water bath, and next, 4.4 g (0.037 mol) of 2, 2-dimethylpropionyl chloride were added. Once the addition was completed, the resultant mixture was stirred at about 0°C for 30 minutes and next, at room temperature for 8 hours. Finally, 75 mL of ethyl ether were added to the mixture, the insoluble residue was filtered off and the liquid was washed with H₂O (2 x 100 mL) . The organic phase was dried over anhydrous Na₂SO₄, the sovent evaporated under reduced pressure, and the product was isolated as a solid with m.p. 231-3°C (recrystallized in ethyl acetate) with a yield of 28%.

| **Quantitative Analysis:** Calculated for C₂₀H₂₂N₂O₂ | | | |
|---|---|---|---|
| | %C | %H | %N |
| Calculated | 74.51 | 6.88 | 8.69 |
| Found | 74.81 | 6.85 | 8.54 |

### Example 40

### 2,2-Dimethyl-propionic acid 3-(5-nitro-1H-benzimidazol-2-yl)phenyl ester

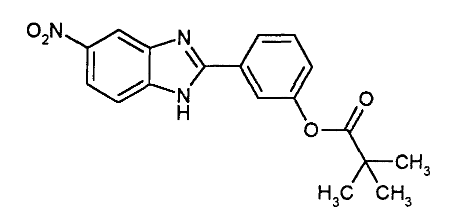

Initially, 14 mL of triethylamine were added dropwise to a stirred solution composed of 10 g (0.039 mol) of 2-(3-hydroxyphenyl)-5-nitrobenzimidazole in 47 mL of anhydrous CH₂Cl₂, using external cooling with an ice-water bath, and next, 4.72 g (0.039 mol) of 2, 2-dimethylpropionyl chloride were added. Once the addition was completed, the resultant mixture was stirred at about O°C for 30 minutes and then, at room temperature for 4 hours. After that, 75 mL of ethyl ether were added to the reaction mixture, the insoluble residue was filtered off and the liquid was washed with H₂O (2 x 100 mL). Then, the organic phase was dried over anhydrous Na₂SO₄, the solvent was evaporated under reduced pressure, and the product was isolated as a solid with m.p. 201-3 °C (recrystallized in methanol) with a yield of 82%.

| **Quantitative Analysis:** Calculated for C₁₈H₁₇N₃O₄ | | | |
|---|---|---|---|
| | % C | % H | % N |
| Calculated | 63.71 | 5.05 | 12.38 |
| Found | 64.00 | 5.12 | 12.28 |

### Example 41

### 2,2-Dimethyl-propionic acid 3-(5-nitro-1H-benzimidazol-2-yl)-4-nitro-phenyl ester

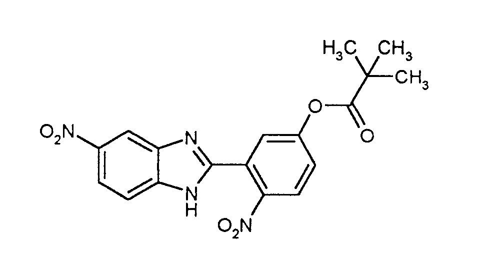

Initially, 7 mL of triethylamine were added dropwise to a stirred solution of 6 g (0.02 mol) of 2-(5-hydroxy-2-nitrophenyl)-5-nitrobenzimidazole in 25 mL of anhydrous CH₂Cl₂, using external cooling with an ice-water bath, and next, 2.41 g (0.02 mol) of 2, 2-dimethylpropionyl chloride were added. Once the addition was completed, the resultant mixture was stirred at about 0°C for 30 minutes and then, at room temperature for 4 hours. After such a time, 50 mL of ethyl ether were added to the mixture, the insoluble residue was filtered off and the liquid was washed with H₂O (2 x 100 ml). Then, the organic phase was dried over anhydrous Na₂SO₄, the solvent evaporated under reduced pressure, and the product was obtained as a solid with m.p. 208-10°C (recrystallized in ethyl acetate) with a yield of 36%.

| **Quantitative Analysis:** Calculated for C₁₈H₁₆N₄O₆ | | | |
|---|---|---|---|
| | % C | % H | % N |
| Calculated | 56.25 | 4.20 | 14.58 |
| Found | 56.42 | 4.17 | 14.53 |

### Example 42

### 2,2-Dimethyl-propionic acid 4-(3H-imidazo[4,5-b]pyridin-2-yl)-phenyl ester

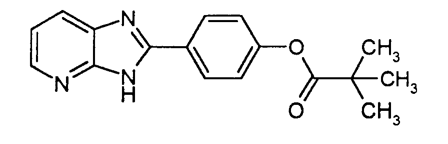

Initially, 18 mL of triethylamine were added dropwise to a stirred solution composed of 10 g (0.047 mol) of 2-(4-hydroxyphenyl)imidazo[4,5-blpyridine in 60 mL of anhydrous CH₂Cl₂, using external cooling with an ice-water bath, and next, 5.71 g (0.047 mol) of 2, 2-dimethylpropionyl chloride were added. Once the addition was completed, the mixture was stirred at about 0°C for 30 minutes and then, at room temperature for 8 hours. After such a time, 60 mL of ethyl ether were added to the reaction mixture, the insoluble residue was filtered off and the liquid was washed with H₂O (2 x 100 mL). The organic phase was dried over anhydrous Na₂SO₄, the solvent evaporated under reduced pressure, and the product was isolated as a solid with m.p. 275-7°C (recrystallized in n-hexane/ethyl acetate 1:1) with a yield of 39%.

| **Quantitative Analysis:** Calculated for C₁₇H₁₇N₃O₂ | | | |
|---|---|---|---|
| | %C | % H | %N |
| Calculated | 69.14 | 5.80 | 14.23 |
| Found | 69.49 | 5.79 | 14.16 |

### Example 43

### 2,2-Dimethyl-propionic acid 4-(3H-imidazo[4,5-b]pyridin-2-yl)-2-methoxy-phenyl ester

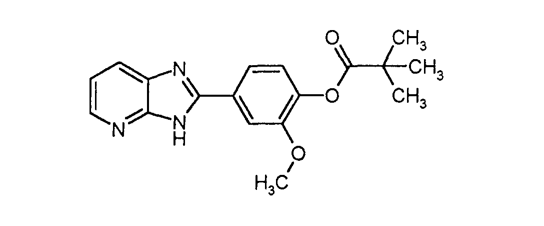

Initially, 16.5 mL of triethylamine were added dropwise to a stirred solution of 10 g (0.041 mol) of 2-(4-hydroxy-3-methoxyphenyl)imidazo[4,5-b]pyridine in 55 mL of anhydrous CH₂Cl₂, using external cooling with an ice-water bath, and next, 4.99 g (0.041 mol) of 2, 2-dimethylpropionyl chloride were added. Once the addition was completed, the mixture was stirred at about 0°C for 30 minutes and, the, at room temperature for 12 hours. After that, 55 mL of ethyl ether were added to the mixture, the insoluble residue was filtered off and the remainig liquid was washed with H₂O (2 x 100 mL). Then, the organic phase was dried over Na₂SO₄, the solvent evaporated under reduced pressure, and the product obtained as a solid with m.p. 255-7°C (recrystallized in methanol) with a yield of 38 %.

| **Quantitative Analysis:** Calculated for C₁₈H₁₉N₃O₃ | | | |
|---|---|---|---|
| | %C | %H | %N |
| Calculated | 66.45 | 5.89 | 12.92 |
| Found | 66.63 | 6.00 | 12.91 |

### Example 44

### 2,2-Dimethyl-propionic acid 2-(3H-imidazo[4,5-b]pyridin-2-yl)-phenyl ester

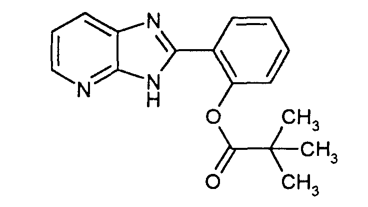

Initially, 18 mL of triethylamine were added dropwise to a stirred solution of 10 g (0.047 mol) of 2-(2-hydroxyphenyl)imidazo[4,5-blpyridine in 60 mL of anhydrous CH₂Cl₂, using external cooling with an ice-water bath, and next, 5.71 g (0.047 mol) of 2, 2-dimethylpropionyl chloride were added. Once the addition was completed, the mixture was stirred at about 0°C for 30 minutes and then, at room temperature for 8 hours. Finally, 60 mL of ethyl ether were added to the reaction mixture, the insoluble residue was filtered and the remaining liquid was washed with H₂O (2 x 100 ml) The organic phase was dried over anhydrous Na₂SO₄, the solvent removed under reduced pressure, and the product isolated as a solid with m.p. 162-4°C (recrystallized in n-hexane/ethyl acetate 1:1) with a yield of 81%.

| **Quantitative Analysis:** Calculated for C₁₇H₁₇N₃O₂ | | | |
|---|---|---|---|
| | % C | % H | % N |
| Calculated | 69.14 | 5.80 | 14.23 |
| Found | 69.29 | 5.85 | 14.17 |

### Example 45

### 2,2-Dimethyl-propionic acid 3-(3H-imidazo[4,5-b]pyridin-2-yl)-phenyl ester

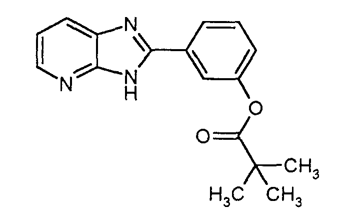

Initially, 14 mL of triethylamine were added dropwise to a stirred solution of 8 g (0.038 mol) of 2-(3-hydroxyphenyl)imidazo[4,5-b]pyridine in 48 mL of anhydrous CH₂Cl₂, using external cooling with an ice-water bath, and then, 4.57 g (0.038 mol) of 2, 2-dimethylpropionyl chloride were added. Once the addition was completed, the resultant mixture was stirred at about 0°C for 30 minutes and then, at room temperature for 9 hours. At the end, 48 mL of ethyl ether were added to the mixture, the insoluble residue was filtered off and the liquid was washed with H₂O (2 x 200 mL). The organic phase was dried over anhydrous Na₂SO₄, the solvent removed under reduced pressure, and the product was obtained as a solid with m.p. 204-6°C (recrystallized in n-hexane/ethyl acetate 1:1) with a yield of 57%.

| **Quantitative Analysis:** Calculated for C₁₇H₁₇N₃O₂ | | | |
|---|---|---|---|
| | % C | % H | % N |
| Calculated | 69.14 | 5.80 | 14.23 |
| Found | 69.54 | 5.82 | 14.40 |

### Example 46

### 2,2-Dimethyl-propionic acid 4-(3H-imidazo[4,5-c]pyridin-2-yl)-phenyl ester

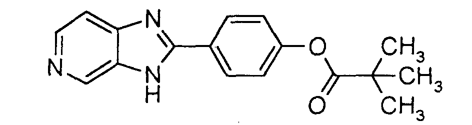

Initially, 10.5 mL of triethylamine were added dropwise to a stirred solution composed of 6 g (0.028 mol) of 2-(4-hydroxyphenyl)imidazo[4,5-c]pyridine in 35 mL of anhydrous CH₂Cl₂, using external cooling with an ice-water bath, and next, 3.43 g (0.028 mol) of 2, 2-dimethylpropionyl chloride. Once the addition was complete, the resultant mixture was stirred at about O °C for 30 minutes and next, at room temperature for 12 hours. At the end, 60 mL of ethyl ether were added to the reaction mixture, the insoluble residue was filtered off, and the liquid was washed with H₂O (2 x 100 ml). Then, the organic phase was dried over anhydrous Na₂SO₄, the solvent was evaporated under reduced pressure, and the product was obtained as a solid with m.p. 240-2 °C (recrystallized in ethyl acetate) with a yield of 60%.

| **Quantitative Analysis:** Calculated for C₁₇H₁₇N₃O₂ | | | |
|---|---|---|---|
| | % C | % H | % N |
| Calculated | 69.14 | 5.80 | 14.23 |
| Found | 68.65 | 6.18 | 13.87 |

### Example 47

### 2,2-Dimethyl-propionic acid 4-(3H-imidazo[4,5-c]pyridin-2-yl)-2-methoxy-phenyl ester

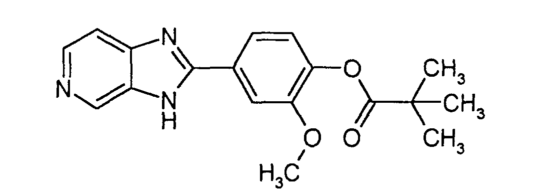

Initially, 15 mL of triethylamine were added dropwise to a stirred solution composed of 6 g (0.025 mol) of 2-(4-hydroxy-3-methoxyphenyl)imidazo[4,5-c]pyridine in 50 mL of anhydrous CH₂Cl₂, using external cooling with an ice-water bath, and then, 4.49 g (0.037 mol) of 2, 2-dimethylpropionyl chloride were added. Once the addition was completed, the mixture was stirred at about 0°C for 30 minutes and next, at room temperature for 4 hours. After such a time, 50 mL of ethyl ether were added to the mixture, the insoluble residue was filtered and the remaining liquid was washed with H₂O (2 x 100 mL). Then, the organic phase was dried over anhydrous Na₂SO₄, the solvent was evaporated under reduced pressure, and the product was obtained as a solid with m.p. 215-7°C (recrystallized in ethyl acetate) with a yield of 59%.

| **Quantitative Analysis:** Calculated for C₁₈H₁₉N₃O₃ | | | |
|---|---|---|---|
| | % C | % H | % N |
| Calculated | 66.45 | 5.89 | 12.92 |
| Found | 66.12 | 5.86 | 12.55 |

### Example 48

### 2,2-Dimethyl-propionic acid 3-(3H-imidazo[4,5-c]pyridin-2-yl)-phenyl ester

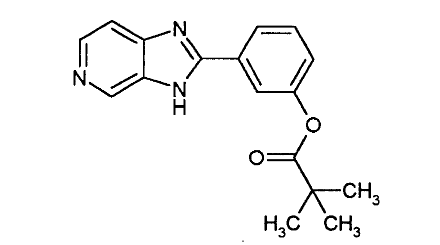

Initially, 12.5 mL of triethylamine were added dropwise to a stirred solution of 7 g (0.033 mol) of 2-(3-hydroxyphenyl)imidazo[4,5-clpyridine in 41 mL of anhydrous CH₂Cl₂, using external cooling with an ice-water bath, and next, 3.99 g (0.033 mol) of 2, 2-dimethylpropionyl chloride were added. Once the addition was completed, the mixture was stirred at about O°C for 30 minutes and next, at room temperature for 8 hours. Then, 75 mL of ethyl ether were added to the reaction mixture, the insoluble residue was filtered off and the remaining liquid was washed with H₂O (2 x 100 ml) . The organic phase was dried over anhydrous Na₂SO₄, the solvent evaporated under reduced pressure, and the product was isolated as a solid with m.p. 246-8°C (recrystallized in diisopropyl ether) with a yield of 69%.

| **Quantitative Analysis:** Calculated for C₁₇H₁₇N₃O₂ | | | |
|---|---|---|---|
| | % C | % H | % N |
| Calculated | 69.14 | 5.80 | 14.23 |
| Found | 68.97 | 5.87 | 14.78 |

### Example 49

### 2,2-Dimethylpropionic acid 4-(5-methyl-5H-imidazo[4,5-c]pyridin-2-yl) phenyl ester. (MAH-5)

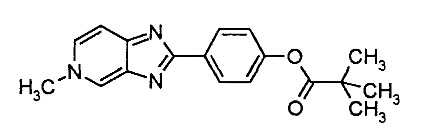

To a stirred solution of the 2,2-dimethyl-propionic acid 4-(1H-imidazo[4,5-c]piridin-2-yl)-phenyl ester in acetone (20 ml) was added methyl iodide (5 ml). The mixture was stirred to reflux 18 h. Then the solvent was concentrated under reduced pressure, and the product was triturated and filtrated with EtOAc. The solid was purified by column chromatography on silica gel , eluting with CH₂Cl₂ / MeOH (10/1) to give a white solid, with a melting point of 208-209 °C. Yield: 80%.

| **Quantitative Analysis** Calculated for C₁₈H₁₉N₃O₂ (309.37 g/mol): | | | |
|---|---|---|---|
| | %C | %H | %N |
| Calculated | 69.88 | 6.19 | 13.58 |
| Found | 69.57 | 5.98 | 13.26 |

### Example 50

### 2,2-Dimethylpropionic acid 4-(5-ethyl-5H-imidazo[4,5-c]pyridin-2-yl) phenyl ester, hydrogen oxalate. (MAH-9)

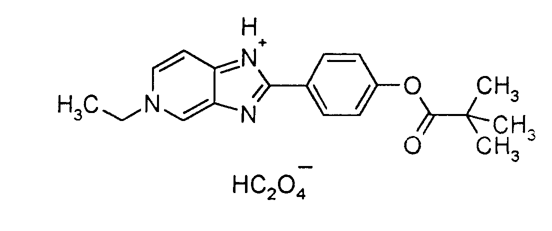

To a stirred solution of the 5-ethyl-2-(4-hydroxy-phenyl)-1H-imidazo[4,5-c]piridin-5-ium bromide (0.80 g, 2.2 mmol) and NaOH (0.44 g, 10.9 mmol) in dry CH₂Cl₂ (50 mL) at room temperature was added pivaloyl chloride (0.52 g, 4.4 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel, eluting with CH₂Cl₂ / MeOH (20/1) to give an oil, which was isolated as oxalate. The salt was recrystallized from EtOH, giving a melting point of 198-199 °C.Yield: 62%

| **Quantitative Analysis:** Calculated for C₂₁H₂₃N₃O₆ (413.43 g/mol): | | | |
|---|---|---|---|
| | %C | %H | %N |
| Calculated | 61.01 | 5.61 | 10.16 |
| Found | 60.89 | 5.56 | 10.32 |

### Example 51

### 2,2-Dimethylpropionic acid 4-(5-benzyl-5H-imidazo[4,5-c]pyridin-2-yl)phenyl ester. (MAH-6)

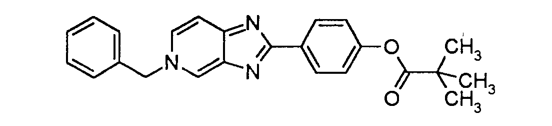

To a stirred solution of the 2,2-dimethyl-propionic acid 4-(1H-imidazo[4,5-c]piridin-2-yl)-phenyl ester in acetone ( 20 ml ) was added methyl iodide (5 ml). The mixture was stirred to reflux 18 h. Then, the solvent was concentrated under reduced pressure, and the product was triturated and filtrated with EtOAc. The solid was purified by column chromatography on silica gel , eluting with CH₂Cl₂/ MeOH (10/1) to give a white solid, with a melting point of 227-228 °C. Yield: 80%.

| **Quantitative Analysis:** Calculated for C₂₄H₂₃N₃O₂ (385.47 g/mol): | | | |
|---|---|---|---|
| | %C | %H | %N |
| Calculated: | 74.78 | 6.01 | 10.90 |
| Found: | 74.59 | 6.09 | 10.96 |

### Example 52

### 2,2-Dimethylpropionic acid 4-[5-(2-piperidin-1-yl ethyl)-5H-imidazo[4,5-c]pyridin-2-yl] phenyl ester. (MAH-11)

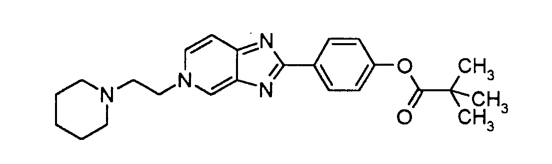

To a stirred solution of the 5-(2-piperidin-1-yl-ethyl)-2-(4-hydroxy-phenyl)-1H-imidazo[4,5-c]piridin-5-ium bromide (2.0 g, 6.2 mmol) and NaOH (1.25 g, 30.9 mmol) in dry CH₂Cl₂ (50 mL) at room temperature was added pivaloyl chloride (1.52 g, 12.4 mmol). The mixture was stirred for 5 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel , eluting with acetone / MeOH (10/1) to give a white solid, which was recrystallized from Et₂O, giving a melting point of 207-208 °C.Yield: 36%

| **Quantitative Analysis:** Calculated for C₂₄H₃₀N₄O₂ (406.53 g/mol): | | | |
|---|---|---|---|
| | %C | %H | %N |
| Calculated | 70.91 | 7.44 | 13.78 |
| Found | 70.65 | 7.42 | 13.97 |

### Example 53

### 2,2-Dimethylpropionic acid 4-[5-(2-piperidin-1-yl propyl)-5H-imidazo[4,5-c]pyridin-2-yl] phenyl ester, dihydrogen oxalate. (MAH-12)

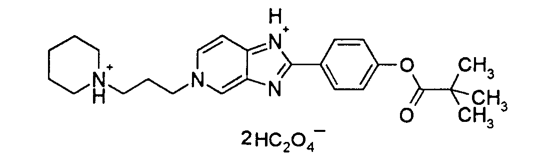

4-(imidazo[4,5-c]pyridin-2-yl)phenol (0.5 g, 2.36 mmol) and 1-(3-iodopropyl)piperidine (0.9 g, 3.55 mol) in CH₃CN at reflux was stirred for 24 h. Then the mixture was concentrated under reduced pressure. To a stirred solution of the residue and NaOH ( 0.47 g, 11.8 mmol) in dry CH₂Cl₂ (50 mL) at room temperature was added pivaloyl chloride (0.28 g, 4.72 mmol). The mixture was stirred for 24 h and then H₂O (50 mL ) was added. The organic layer was separated and the aqueous layer was extracted with CH₂Cl₂ (2x25 ml). The combined organic layers were dried over Na₂SO₄ and concentrated under reduced pressure. The residue was purified by column chromatography on silica gel, eluting with acetone / MeOH (10/1) to give a colourless oil, which was isolated as oxalate. The salt was recrystallized from EtOH, giving a melting point of 189-190 °C. Yield: 19%

| **Quantitative Analysis:** Calculated for C₂₉H₃₆N₄O₁₀ (600.63 g/mol) : | | | |
|---|---|---|---|
| | %H | %C | %N |
| Calculated | 6.04 | 57.99 | 9.33 |
| Found: | 5.78 | 57.72 | 9.58 |

As examples 54 and 55 are more elaborated, and not simply obtained from the phenolic precursor, the full synthesis is described for both compounds.

### Example 54

### 2, 2-dimethylpropionic acid 4-[5-(3-{4-[Bis-(4-fluoro-phenyl)-methyl]-piperazin-1-yl}-ethyl)-5H-imidazo[4,5-c]pyridin-2-yl]-phenyl-ester (12).

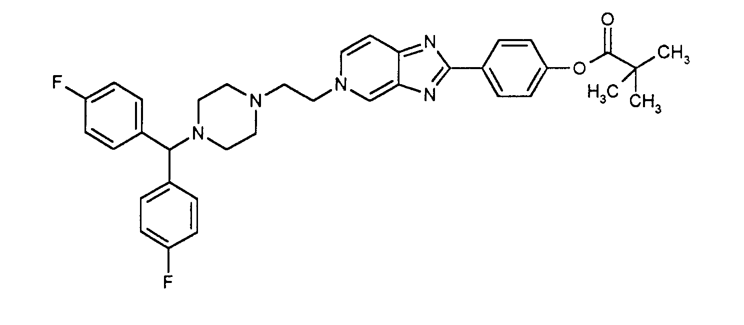

1) 4-(3H-lmidazo[4,5-c]pyridin-2-yl)-phenol **(3)**(Scheme 1). To equivalent amounts (500mg, 4.58 mmol) of 3,4-diaminopyridine and 4-hidroxybenzaldehyde (559mg) 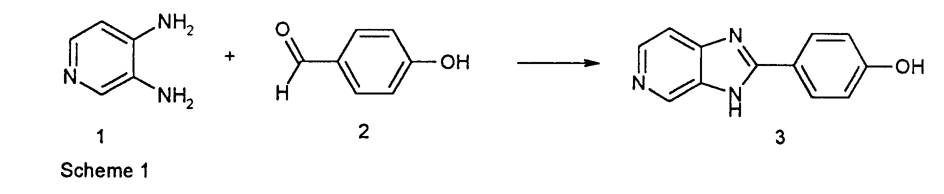 in MeOH (10 mL ), SiO₂ (2.5g) was added. The solvent was evaporated to dryness and the resultant mixture was subjected to microwave irradiation in a domestic microwave oven for ten minutes (550W). The product was purified by silica gel chromatography, being eluted with CH₂Cl₂/MeOH (8:2). Compound 3 was obtained as a yellow solid (73%) with a m.p.= 246-8 °C
2) 1-[bis-(4-fluorophenyl)-methyl]-piperazine (6)(Scheme 2). To a stirred solution of 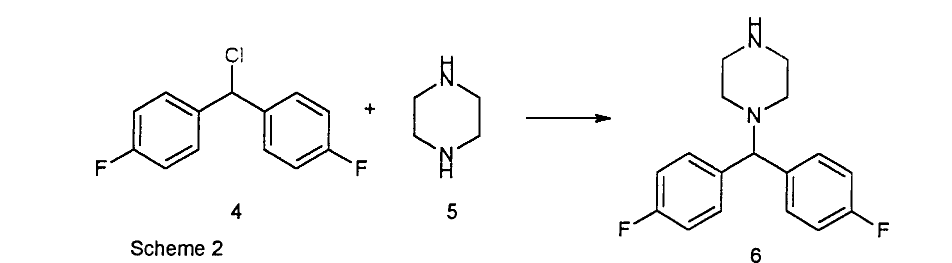 **4** (480mg, 2 mmol ) in DMSO (10 mL), piperazine **5** (860mg, 10 mmoles) and Kl (100mg, 0.5 mmol) in the same solvent (10 mL) was added. Triethylamine (1.4ml, 10 mmol) was added dropwise and the mixture was refluxed for 48 hours. The reaction mixture is poured into saturated solution of NaHCO₃ and extracted with ether (3x50 mL). The combined organic phases were dried over Na₂SO₄, filtered, and evaporated to dryness. The product was purified by silica gel chromatography, being eluted with hexane/ether (3:1) yielding the compound **6** like a white solid (86%) with a m.p = 90-1°C (Lit. 90-93 °C; S. Gubert, M. Brasó, A. Sacristan, J. Ortiz; *Arzneim. Forsh.* **1987**,, 37(*II*), 1103) 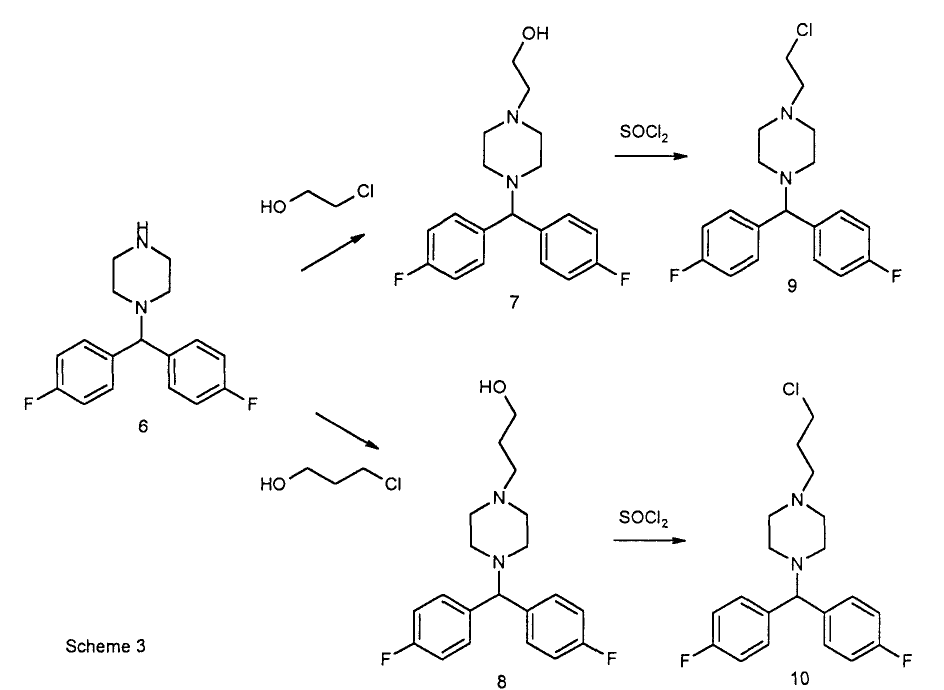
3) 2-(4-[Bis-(4-fluoro-phenyl)-methyl]-piperazin-1-yl)-ethanol (**7**)(Scheme 3). To a stirred solution of **6** (200mg, 0.69mmol) in acetonitrile (5mL), K₂CO₃ (143mg, 1.03 mmol) was added. Afterwards, 2-bromoethanol (94.9mg, 0.76 mmol) was added dropwise. The mixture was refluxed for 23 hours. The inorganic precipitate was filtered off and the solvent evaporated to dryness. The product was purified by silica gel chromatography, being eluted with ethyl acetate/methanol (4:1) yielding the compound 7 (72%) as a yellow oil.
**4)** 1-[Bis-(4-fluoro-phenyl)-methyl]-4-(2-chloroethyl)-piperazine (**9**)(Scheme 3). The compound **7** (3.44g, 10.35 mmol) in thionyl chloride (3.69g, 31.05 mmol) was refluxed for 1/2 hour. The reaction mixture was made basic with NaOH (10%) and extracted with CH₂Cl₂. The organic phase was dried over Na₂SO₄, filtered and evaporated to dryness. The product was purified by silica gel chromatography, being eluted with ethyl acetate to afford 9 ( 85 %) as an oil.
**5)** 4-[5-(2-{4-[Bis-(4-fluoro-phenyl)-methyl]-piperazin-1-yl}-ethyl)-5H-imidazo[4,5-c]pyridin-2-yl]-phenol (**11**)(Scheme 4). Equivalent amounts (8.63 mmol) of compound **3** (1.82g) and compound **9** (3.03g) were dissolved in DMF (90 mL). The reaction mixture was refluxed for 19 hours. The organic solvent was evaporated to dryness. Purification of the reaction mixture by column chromatography on silica gel (ethyl acetate/methanol 4:1) yielded compound **11** in 44% yield as a yellow solid with a m. p.= 176-7°C.

| **Quantitative Analysis:** Calculated for C₃₆H₃₇N₂O₂F₂ | | | |
|---|---|---|---|
| | % C | % H | % N |
| Calculated | 76.17 | 6.57 | 4.93 |
| Found | 76.34 | 6.37 | 4.71 |

**6)** 4-[5-(3-{4-[bis-(4-fluoro-phenyl)-methyl]-piperazin-1-yl}-ethyl)-5H-imidazo[4,5-c]pyridin-2-yl]-phenyl 2, 2-dimethylpropionate.(**12**)(Scheme 4). The compound 11 (1.28g, 2.4 mmol) was dissolved in DMF (50 ml).The solution was heated at 60 °C and NaOH (0.18g, 4.5 mmol) was added. The solution was stirred a few minutes and afterwards pivaloyl chloride (0.54g, 4.5 mmol) was added dropwise. The mixture was refluxed for 18 hours. The reaction mixture was poured in water and extracted with CH₂Cl₂. The combined organic phases were dried over Na₂SO₄, filtered and evaporated to dryness. The product was purified by silica gel chromatography (ethyl acetate/methanol 4:1). The compound 12 was isolated as hydrochloride (76%) with a m.p. = 204-6 °C.

| **Quantitative Analysis:** Calculated for C₃₁H₂₉N₂OF₂ | | | |
|---|---|---|---|
| | %C | %H | %N |
| Calculated | 77.00 | 6.04 | 5.79 |
| Found | 76.84 | 6.32 | 5.71 |

### Example 55

### 2,2-Dimethyl-propionic acid 4-[5-(3-{4-[bis-(4-fluoro-phenyl)-methyl]-piperazin-1-yl}-propyl)-5H-imidazo[4,5-c]pyridin-2-yl]-phenyl ester (14).

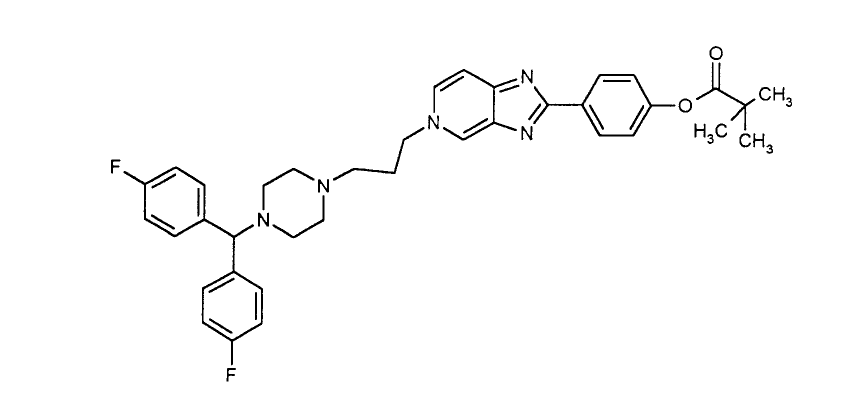

**1)** 3-(4-[Bis-(4-fluoro-phenyl)-methyl]-piperazin-1-yl)-propan-1-ol (**8**)(Scheme 3). To a stirred solution of **6** (9.59g, 33.25 mmol) in acetonitrile (300 mL), K₂CO₃ (6.43 g, 46.55 mmol) was added. Afterwards 3-bromopropanol (5.09g, 36.6 mmol) was added dropwise. The mixture was refluxed for 15 hours. The inorganic precipitate was filtered and the organic solvent was evaporated to dryness. The product was purified by silica gel chromatography, being eluted with ethyl acetate/methanol (4:1) yielding the compound 8 (72%) as an oil.
**2)** 1-[Bis-(4-fluorophenyl)methyl]-4-(3-chloropropyl)piperazine (**10**)(Scheme 3). The compound **8** (8.27g, 24 mmol) in thionyl chloride (5.71g, 72 mmol) was refluxed for 1 hour. The reaction mixture was made basic with NaOH (10%) and extracted with CH₂Cl₂. The organic phase was dried over Na₂SO₄, filtered and evaporated to dryness. The product was purified by silica gel chromatography, being eluted with ethyl acetate to afford 10 (49 %) as an oil. 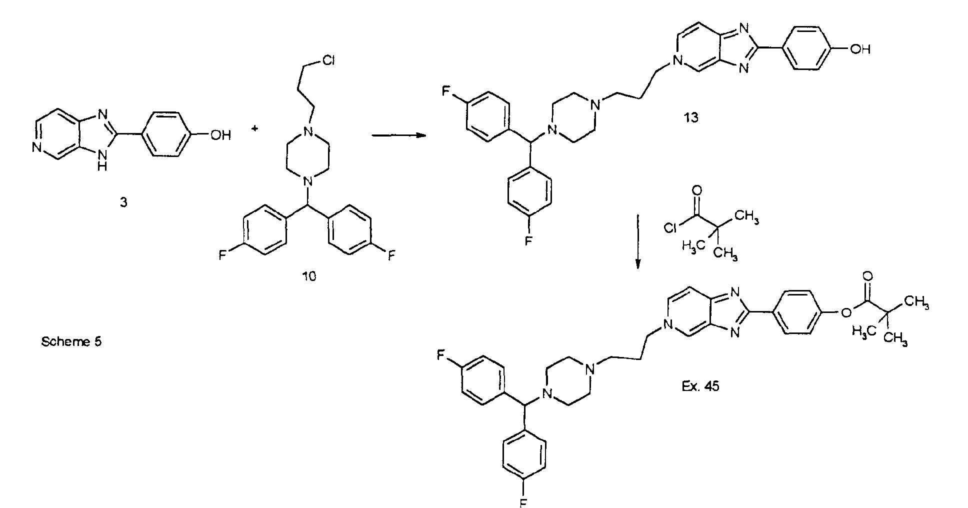
**3)** 4-[5-(3-{4-[Bis-(4-fluoro-phenyl)-methyl]-piperazin-1-yl}-propyl)-5H-imidazo[4,5-c]pyridin-2-yl]-phenol (**13**)(Scheme 5). The compound **3** (1.8g, 8.52 mmol) and compound 10 (3.87g, 7.17 mmol) were dissolved in DMF (80 mL). The reaction mixture was refluxed for 19 hours. The organic solvent was evaporated to dryness. Purification of the reaction mixture by column chromatography on silica gel (ethyl acetate/methanol 4:1) yielded compound **13** in 43% yield as a yellow solid with a m. p.: 240-3 °C.

| **Quantitative Analysis:** Calculated for C₃₂H₃₁N₂OF₂ | | | |
|---|---|---|---|
| | % C | % H | % N |
| Calculated | 77.24 | 6.28 | 5.63 |
| Found | 77.34 | 6.17 | 5.84 |

4) 2,2-Dimethyl-propionic acid 4-[5-(3-{4-[bis-(4-fluoro-phenyl)-methyl]-piperazin-1-yl}-propyl)-5H-imidazo[4,5-c]pyridin-2-yl]-phenyl ester (14). The compound 13 (1.63g, 3.02 mmol) was dissolved in DMF (50 ml).The solution was heated at 60°C and NaOH (0.36g, 9.06 mmol) was added. The solution was stirred a few minutes and afterwards pivaloyl chloride (1.09g, 9.06 mmol) was added dropwise. The mixture was refluxed for 18 hours. The reaction mixture was poured in water and extracted with CH₂Cl₂. The combined organic phases were dried over Na₂SO₄, filtered and evaporated to dryness. The product was purified by silica gel chromatography, (Cl₂CH₂/methanol 9:0.5). The compound 14 was isolated as hydrochloride. (54 %) with a m. p.= 199-201 °C.

| **Quantitative Analysis:** Calculated for C₃₇H₃₉N₂O₂F₂ | | | |
|---|---|---|---|
| | % C | % H | % N |
| Calculated | 76.39 | 6.76 | 4.82 |
| Found | 76.34 | 6.87 | 4.64 |

### Example 56

### 2,2-Dimethyl-propionic acid 4-(1-H-benzimidazol-2-yl)-2,3-bis-(2,2-dimethyl-propionyloxy)-phenyl ester

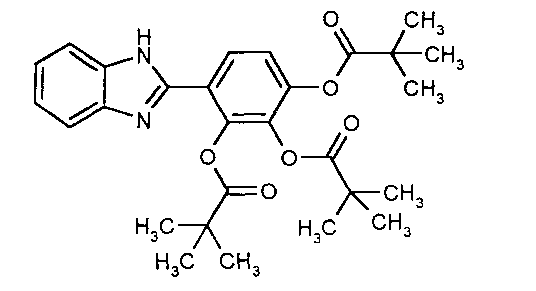

Initially, 13.5 mL of triethylamine were added dropwise to a stirred solution composed of 8.71 g (0.036 mol) of 2-(2,3,4-trihydroxyphenyl)benzimidazole in 45 mL of anhydrous CH₂Cl₂, using external cooling with an ice-water bath, and next, 17.28 g (0.144 mol) of 2, 2-dimethylpropionyl chloride were added. Once the addition was completed, the resultant mixture was stirred at about 0°C for 30 minutes, and then, at room temperature for 4 hours. After that, 75 mL of ethyl ether were added to the mixture, the insoluble residue was filtered off, and the liquid was washed with H₂O (2 x 100 ml). Then, the organic phase was dried over anhydrous Na₂SO₄, the solvent was evaporated under reduced pressure, and the product was obtained as a solid with m.p. 172-4°C (recrystallized in methanol) with a yield of 70%.

| **Quantitative Analysis:** Calculated for C₂₈H₃₄N₂O₆. | | | |
|---|---|---|---|
| | % C | % H | % N |
| Calculated | 68.00 | 6.93 | 5.66 |
| Found | 68.35 | 6.80 | 5.82 |

### Pharmacological and toxicological tests:

The pharmacological activity of the compounds of formula (I) according to the invention have been verified through the following biological tests, for some of said compounds.
The method employed was based on that described by Bieth, J., Spiess, B. and Wermuth, C.G. (1974), Biochem. Med. 11; 350-357 with some modifications.
The hydrolytic activity of HLE (Sigma, Deisenhofen, Germany) on the peptide substrate MeO-Suc-Ala-Ala-Pro-Val-p-nitroanilide (Sigma) was measured in 96-well F-botton microliter plates. The assay buffer used consisted of 50mM Tris-HCI (pH 8) with 50mM NaCI and 0.01% Brij 35.
The enzyme (0.2 U/ml; 50µl) was preincubated for 15 min at mom temperature in the presence of test compounds or vehicle (DMSO) in a total volume of 100 µl.
The reaction was started by addition of 50 µl substrate (0.5mM) and formation of p-nitroanilid was monitored by detection at 406 nm for 10 min.
Percent inhibition of enzyme activity was calculated in comparison to the corresponding vehicle control and the results obtained are mentioned in the following Table.

**Table**

| **Exemple n°** | **IC50 IN M** |
|---|---|
| 37 | 5.90^{E}-08 |
| 21 | 6.00^{E}-08 |
| 15 | 6.90^{E}-08 |
| 13 | 8.80^{E}-08 |
| 20 | 1.00^{E}-07 |
| 42 | 1.35^{E}-07 |
| 41 | 1.82^{E}-07 |
| 46 | 2.37^{E}-07 |
| 17 | 2.90^{E}-07 |
| 1 | 3.06^{E}-07 |
| 18 | 3.3^{E}-7 |
| 5 | 3.83^{E}-7 |
| 45 | 4.74^{E}-7 |
| 10 | 4.86^{E}-7 |
| 48 | 6.09^{E}-7 |
| 50 | 6.1^{E}-7 |
| 52 | 6.25^{E}-7 |
| 40 | 6.78^{E}-7 |
| 9 | 6.82^{E}-7 |
| 56 | 7.08^{E}-7 |
| 35 | 1.26^{E}-6 |
| 43 | 1.66^{E}-6 |
| 22 | 1.7^{E}-5 |
| 27 | 1.52^{E}-5 |

Regarding the toxicity it is stated that the most active compounds of formula (I) according to the invention present a low per oral toxicity with LD₅₀ more than 500 mg/kg in mice.

## Claims

1. Esters of 2,2-dimethylpropionic acid having the general formula (I): 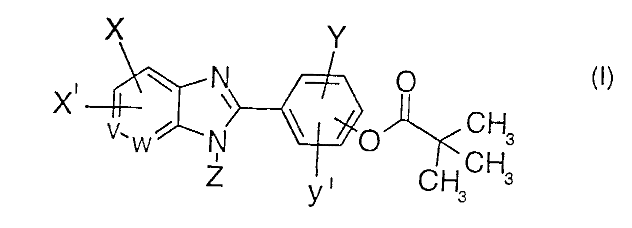 or a pharmacological acceptable salt thereof, where
x and x' represent a hydrogen atom, an alkyl group in C1-C4, an halogen atom or a group nitro;
y and y' represent a hydrogen atom, a group alkyl in C1-C4, a group alkoxy in C1-C4, an halogen atom or a group dialkyl(C1-C4)amino;
z represents a hydrogen atom, a dialkyl(C1-C4)aminoalkyl(C1-C4) group or a piperidinyl-alkyl(C1-C4) group; and
v and w represent a carbon atom bound to a hydrogen atom (CH) or a nitrogen atom substituted or not.

2. Compounds of formula (I) according to claim 1, where
x and/or x' represent the group methyl or nitro, or a chlorine atom;
y and/or y' represent the group methyl, methoxy, nitro or diethylamino, or a chlorine, a bromine or a fluorine atom; and
z represents a group dimethylaminoethyl, dimethylaminopropyl, diisopropylaminoethyl or piperidinyl-ethyl.

3. Compounds of formula (I) according to claim 1, where v or w represents a nitrogen atom substituted by a group methyl, ethyl, benzyl, piperidinyl-ethyl, piperidinyl-propyl, bis(fluorophenyl)methyl-piperazinyl-ethyl or bis(fluorophenyl) methyl-piperazinyl-propyl.

4. The following compounds of formula (I) according to claim 1,
2,2-Dimethyl-propionic acid 4-(1H-benzimidazol-2-yl)-phenyl ester
2,2-Dimethyl-propionic acid 4-(1H-benzimidazol-2-yl)-2-ethoxy-phenyl ester
2,2-Dimethyl-propionic acid 4-(1H-benzimidazol-2-yl)-2,6-dimethoxy-phenyl ester
2,2-Dimethyl-propionic acid 4-(1H-benzimidazol-2-yl)-2-chloro-phenyl ester
2,2-Dimethyl-propionic acid 4-(1H-benzoimidazol-2-yl)-2-nitro-phenyl ester
2,2-Dimethyl-propionic acid 4-(1H-benzimidazol-2-yl)-2-nitro-6-methoxy-phenyl ester
2,2-Dimethyl-propionic acid 4-(5-chloro-1 H-benzimidazol-2-yl)phenyl ester
2,2-Dimethyl-propionic acid 4-(5-chloro-1 H-benzimidazol-2-yl)-2-methoxy-phenyl ester
2,2-Dimethyl-propionic acid 4-(5,6-dimethyl-1H-benzimidazol-2-yl)phenyl ester
2,2-Dimethyl-propionic acid 4-(5-methyl-1 H-benzimidazol-2-yl)phenyl ester
2,2-Dimethyl-propionic acid 4-(5-methyl-1 H-benzimidazol-2-yl)-2-methoxy-phenyl ester
2,2-Dimethyl-propionic acid 4-(5,6-dimethyl-1H-benzimidazol-2-yl)-2-methoxyphenyl ester
2,2-Dimethyl-propionic acid 4-(5-nitro-1 H-benzimidazol-2-yl)phenyl ester
2,2-Dimethyl-propionic acid 4-(5-nitro-1 H-benzimidazol-2-yl)-6-methoxy-2-nitrophenyl ester
2,2-Dimethylpropionic acid 4-[1-(2-dimethylaminoethyl)-1 H-benzimidazol-2-yl] phenyl ester.
2,2-Dimethylpropionic acid 2-bromo-4-[1-(2-dimethylaminoethyl)-1H-benzimidazol2-yl]phenyl ester
2,2-Dimethylpropionic acid 4-[1 -(2-dimethylaminopropyl)-1H-benzimidazol-2-yl]phenyl ester, dihydrogen oxalate
2,2-Dimethylpropionic acid 4-[1-(2-diisopropylaminoethyl)-1H-benzimidazol-2-yl]phenyl ester.
2,2-Dimethylpropionic acid 4-[5,6-dichloro-1-(2-dimethylaminoethyl) 1H-benzimidazol-2-yl] phenyl ester
2,2-Dimethylpropionic acid 4-[5,6-dimethyl-3-(2-piperidin-1-yl-ethyl)-1H-benzimidazol-2-yl] phenyl ester
2,2-Dimethylpropionic acid 2-fluoro-4-[1-(2-piperidin-1-yl ethyl)-1H-benzimidazol-2-yl] phenyl ester
2,2-Dimethyl-propionic acid 2-(1H-benzimidazol-2-yl)phenyl ester
2,2-Dimethyl-propionic acid 2-(1H-benzimidazol-2-yl)-4-chloro-phenyl ester
2,2-Dimethyl-propionic acid 2-(1H-benzimidazol-2-yl)-5-chloro-phenyl ester
2,2-Dimethyl-propionic acid 2-(1H-benzimidazol-2-yl)-4,6-dichloro-phenyl ester
2,2-Dimethyl-propionic acid 2-(1H-benzimidazol-2-yl)-6-methoxy-phenyl ester
2,2-Dimethyl-propionic acid 2-(5-chloro-1H-benzimidazol-2-yl)phenyl ester
2,2-Dimethyl-propionic acid 2-(-5-chloro-1H-benzimidazol-2-yl)-5-diethylaminophenyl ester
2,2-Dimethyl-propionic acid 2-(5,6-dimethyl-1H-benzimidazol-2-yl)phenyl ester
2,2-Dimethyl-propionic acid 2-(5-methyl-1H-benzimidazol-2-yl)-4-chloro-phenyl ester
2,2-Dimethyl-propionic acid 2-(5,6-dimethyl-1H-benzimidazol-2-yl)-diethylaminophenyl ester
2,2-Dimethyl-propionic acid 2-(5-nitro-1H-benzimidazol-2-yl)-phenyl ester
2,2-Dimethyl-propionic acid 2-(5-nitro-1H-benzimidazol-2-yl)-4-chloro-phenyl ester
2,2-Dimethyl-propionic acid 2-(5-nitro-1H-benzimidazol-2-yl)-6-methyl-phenyl ester
2,2-Dimethyl-propionic acid 5-(1H-benzimidazol-2-yl)-phenyl ester
2,2-Dimethyl-propionic acid 3-(1H-benzimidazol-2-yl)-6-methoxy-phenyl ester
2,2-Dimethyl-propionic acid 3-(1H-benzimidazol-2-yl)-4-nitro-phenyl ester
2,2-Dimethyl-propionic acid 3-(5-chloro-1H-benzimidazol-2-yl)phenyl ester
2,2-Dimethyl-propionic acid 3-(5,6-dimethyl-1H-benzimidazol-2-yl)phenyl ester
2,2-Dimethyl-propionic acid 3-(5-nitro-1H-benzimidazol-2-yl)phenyl ester
2,2-Dimethyl-propionic acid 3-(5-nitro-1H-benzimidazol-2-yl)-4-nitro-phenyl ester
2,2-Dimethyl-propionic acid 4-(3H-imidazo[4,5-b]pyridin-2-yl)-phenyl ester
2,2-Dimethyl-propionic acid 4-(3H-imidazo[4,5-b]pyridin-2-yl)-2-methoxy-phenyl ester
2,2-Dimethyl-propionic acid 2-(3H-imidazo[4,5-b]pyridin-2-yl)-phenyl ester
2,2-Dimethyl-propionic acid 3-(3H-imidazo[4,5-b]pyridin-2-yl)-phenyl ester
2,2-Dimethyl-propionic acid 4-(3H-imidazo[4,5-c]pyridin-2-yl)-phenyl ester
2,2-Dimethyl-propionic acid 4-(3H-imidazo[4,5-c]pyridin-2-yl)-2-methoxy-phenyl ester
2,2-Dimethyl-propionic acid 3-(3H-imidazo[4,5-c]pyridin-2-yl)-phenyl ester
2,2-Dimethylpropionic acid 4-(5-methyl-5H-imidazo[4,5-c]pyridin-2-yl) phenyl ester.
2,2-Dimethylpropionic acid 4-(5-ethyl-5H-imidazo[4,5-c]pyridin-2-yl) phenyl ester, hydrogen oxalate
2,2-Dimethylpropionic acid 4-(5-benzyl-5H-imidazo[4,5-c]pyridin-2-yl)phenyl ester
2,2-Dimethylpropionic acid 4-[5-(2-piperidin-1-yl ethyl)-5H-imidazo[4,5-c]pyridin-2-yl] phenyl ester
2,2-Dimethylpropionic acid 4-[5-(2-piperidin-1-yl propyl)-5H-imidazo[4,5-c] pyridin-2-dihydrogen oxalate yl] phenyl ester
2, 2-dimethylpropionic acid 4-[5-(3-{4-[bis-(4-fluoro-phenyl)-methyl]-piperazin-1-yl}-ethyl)-5H-imidazo[4,5-c]pyridin-2-yl]-phenyl-ester
2,2-Dimethyl-propionic acid 4-[5-(3-{4-[bis-(4-fluoro-phenyl)-methyl]-piperazin-1-yl}-propyl)-5H-imidazo[4,5-c]pyridin-2-yl]-phenyl ester
2,2-Dimethyl-propionic acid 4-[(1-H-benzimidazol-2-yl)-2,2-dimethyl-propionyloxy]phenyl ester

5. Esters of 2,2-dimethylpropionic acid having the general formula (I) according to claim 1, or a pharmaceutically acceptable salt thereof, as having an inhibitory activity of elastase.

6. Pharmaceutical compositions containing at least one ester of 2,2-dimethylpropionic acid of formula (I) according to claim 1, or a pharmaceutically acceptable salt thereof.

7. Pharmaceutical compositions according to claim 6, in which the quantity of ester of formula (I) is such that the dose level to be administered is comprised between 0,001 and 10 mg/kg.
